# EUROPÄISCHE PATENTSCHRIFT

(11) **EP 2 007 774 B1**
(45) Veröffentlichungstag und Bekanntmachung des Hinweises auf die Patenterteilung: **25.07.2012**
(21) Anmeldenummer: 07727565.9
(22) Anmeldetag: 30.03.2007
(51) Int. Cl.: C07D 513/04, A61K 31/41, A61P 35/00

(54) **THIAZOLYLDIHYDROINDAZOL-DERIVATE ALS PROTEINKINASE- INHIBITOREN**
THIAZOLYLDIHYDROINDAZOLE DERIVATIVES AS PROTEIN KINASE INHIBITORS
DERIVES DE THIAZOLYLDIHYDROINDAZOL UTILISES EN TANT QU'INHIBITEURS DE PROTEINE KINASE

(30) Priorität: 06.04.2006 EP 06112304
(43) Veröffentlichungstag der Anmeldung: 31.12.2008
(73) Patentinhaber: Boehringer Ingelheim International GmbH, 55216 Ingelheim am Rhein (DE)
(72) Erfinder: MCCONNELL, Darryl, A-1130 Vienna (AT); BETZEMEIER, Bodo, A-2721 Bad Fischau (AT); GERSTBERGER, Thomas, A-1230 Vienna (AT); IMPAGNATIELLO, Maria, A-1030 Vienna (AT); STEURER, Steffen, A-1190 Vienna (AT); VAN DER VEEN, Lars, A-1230 Vienna (AT); WEYER-CZERNILOFSKY, Ulrike, A-2500 Baden (AT)
(74) Vertreter: Hammann, Heinz
(86) Internationale Anmeldenummer: PCT/EP2007/053093
(87) Internationale Veröffentlichungsnummer: WO 2007/113246

(56) Entgegenhaltungen:
- WO-A-01/57008
- WO-A-2006/040281

## Beschreibung

Die vorliegende Erfindung betrifft neue Thiazolyl-dihydro-indazole der allgemeinen Formel (1) 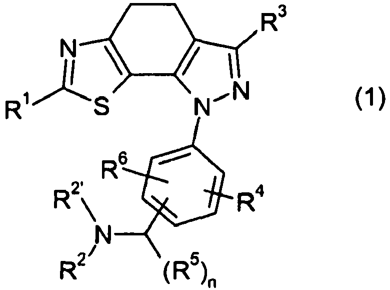 wobei die Reste R¹ bis R⁶ die in den Ansprüchen und der Beschreibung genannten Bedeutungen haben, deren Isomere, Verfahren zur Herstellung dieser Thiazolyl-dihydro-indazole sowie deren Verwendung als Arzneimittel.

### Hintergrund der Erfindung

Eine Reihe von Protein-Kinasen haben sich bereits als geeignete Zielmoleküle für die therapeutische Intervention in verschiedenen Indikationen, z.B. Krebs, entzündlichen und Autoimmunerkrankungen erwiesen. Da ein hoher Prozentsatz der bisher identifizierten, an der Krebsentstehung beteiligten Gene für Kinasen kodiert, stellen diese Enzyme speziell für die Krebstherapie attraktive Zielmoleküle dar.

Phosphatidylinositol-3-Kinasen (PI3-Kinasen) sind ein Subfamilie der Lipid-Kinasen, die die Übertragung eines Phosphatrestes auf die 3'-Position des Inositolrings von Phosphoinositiden katalysieren. Sie spielen eine wichtige Rolle bei zahlreichen zellulären Vorgängen wie z.B. Zellwachstums- und Differenzierungsvorgängen, der Steuerung von zytoskeletalen Veränderungen und der Regulation intrazellulärer Transportvorgänge. Aufgrund ihrer *in vitro*-Spezifität für bestimmte Phosphoinositid-Substrate können die PI3-Kinasen in verschiedene Klassen eingeteilt werden.

Beispielsweise offenbart WO 01/57008 2-Benzothiazolyl-Harnstoff-Derivate, welche als Proteinkinaseinhibitoren verwendet werden können.

### Detaillierte Beschreibung der Erfindung

Es wurde nun überraschenderweise gefunden, dass Verbindungen der allgemeinen Formel (1), worin die Reste R¹ bis R⁶ die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel (1) 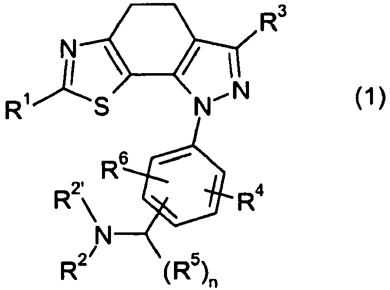 worin
**R¹** ausgewählt aus der Gruppe bestehend aus -NHR^{a}, -NHC(O)R^{a}, -NHC(O)OR^{a}, -NHC(O)NR^{a}R^{a} und -NHC(O)SR^{a}, und
**R²** und **R^{2'}** jeweils unabhängig voneinander Wasserstoff oder ein Rest, ausgewählt aus der Gruppe bestehend aus C₁₋₆Alkyl, C₃₋₈Cycloalkyl, C₆₋₁₀ Aryl 5-10 gliedriges Heteroaryl und 3-8 gliedriges Heterocycloalkyl, gegebenenfalls substituiert mit einem oder mehreren R^{a} und/oder R^{b}, oder
**R²** und **R^{2'}** gemeinsam mit dem eingeschlossenen Stickstoffatom einen Heterocycloalkyl- oder Heteroarylring bilden, welcher gegebenenfalls ein oder mehrere weitere Heteroatome enthalten kann, ausgewählt aus der Gruppe bestehend aus N, O und S und gegebenenfalls mit einem oder mehreren R^{b} und/oder R^{d} substituiert sein kann, und
**R³** ein 5-6 gliedriges Heteroaryl, gegebenenfalls substituiert mit einem oder mehreren, gleich oder verschiedenen R^{c} und/oder R^{b}, und
**R⁴** und **R⁶** jeweils unabhängig voneinander Wasserstoff oder ein Rest ausgewählt aus der Gruppe bestehend aus Halogen, -CF₃, -OCF₃, -CN, -NR^{c}R^{c}, -SR^{c}, -S(O)R^{c}, -S(O)₂R^{c} und - OR^{c}, oder C₁₋₃Alkyl gegebenenfalls substituiert mit Fluor, -CN, -NR^{f}R^{f} und/oder -OR^{f}, und **R⁵** C₁₋₃Alkyl, oder
zwei **R⁵** gemeinsam einen C₃₋₈Cycloalkylring oder 3-8 gliedriges Heterocycloalkyl bilden, und
**n** gleich 0, 1 oder 2, oder
**R²** mit einem **R⁵** einen 4-8 gliedrigen Heterocycloalkylring bilden, oder
**R²** mit einem geeigneten **R⁶** einen 4-8 gliedrigen Heterocycloalkylring bilden, und
jedes **R^{a}** unabhängig voneinander Wasserstoff oder ein gegebenenfalls mit einem oder mehreren, gleich oder verschiedenen R^{b} und/oder R^{c} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und jedes **R^{b}** ein geeigneter Rest jeweils unabhängig voneinander ausgewählt aus der Gruppe bestehend aus =O, -OR^{c}, C₁₋₃Haloalkyloxy, =S, -SR^{c}, =NR^{c}, =NOR^{c}, -NR^{c}R^{c}, Halogen, - CF₃, -CN, -NC, -NO₂, -N₃, -S(O)R^{c}, -S(C)₂R^{c}, -S(O)₂OR^{c}, -S(O)NR^{c}R^{c}, -S(O)₂NR^{c}R^{c}, - OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{d})OR^{c} -CN(R^{d})NR^{c}R^{c}, -OC(O)R^{c}, -OC(O)OR^{c}, -OC(O)NR^{c}R^{c}, -OCN(R^{d})NR^{c}R^{c}, -N(R^{d})C(O)R^{c}, -N(R^{d})C(S)R^{c}, -N(R^{d})S(O)₂R^{c}, -N(R^{d})C(O)OR^{c}, -N(R^{d})C(O)NR^{c}R^{c}, und -N(R^{d})CN(R^{d})NR^{c}R^{c}, und
jedes **R^{c}** unabhängig voneinander ein gegebenenfalls mit einem oder mehreren, gleich oder verschiedenen R^{d} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und
jedes **R^{d}** unabhängig voneinander C₁₋₆Alkyl, C₃₋₈Cycloalkyl, C₄₋₁₁Cycloalkylalkyl, C₆₋₁₀ Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl bedeuten, wobei unter Alkyl-Substituenten jeweils gesättigte, ungesättigte, geradkettige oder verzweigte aliphatische Kohlenwasserstoffreste (Alkylrest) zu verstehen sind und sowohl gesättigte Alkylreste als auch ungesättigte Alkenyl- und Alkinylreste umfasst; und unter Cycloalkyl ein mono- oder bizyklischer Ring zu verstehen ist, wobei das Ringsystem ein gesättigter Ring aber auch ein ungesättigter, nichtaromatischer Ring sein kann, welcher gegebenfalls auch Doppelbindungen enthalten kann ; gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze mit der Maßgabe, dass folgende Verbindungen nicht umfasst sind:
*N-*[1-(4-Morpholin-4-ylmethyl-phenyl)-3-pyridin-2-yl-4,5-dihydro-1*H*-pyrazolo[4,3-*g*]benzthiazol-7-yl]-acetamid,
*N-*[1-(4-Dimethylaminomethyl-phenyl)-3-pyridin-3-yl-4,5-dihydro-1*H*-pyrazolo[4,3-*g*]benzthiazol-7-yl]-acetamid,
*N-*{1-[4-(Benzylamino-methyl)-2-chloro-phenyl]-3-pyrazin-2-yl-4,5-dihydro-1*H-*pyrazolo[4,3-*g*]benzthiazol-7-yl}-acetamid und
*N-*(1-{2-Chloro-4-[(1-cyclopentyl-piperidin-4-ylamino)-methyl]-phenyl}-3-pyrazin-2-yl-4,5-dihydro-1*H*-pyrazolo[4,3-*g*]benzthiazol-7-yl}-acetamid.

Ein Aspekt der Erfindung sind Verbindungen der allgemeinen Formel (1A), 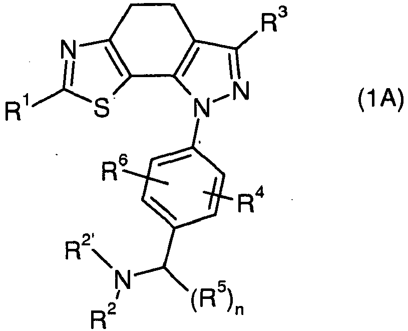 worin die Substituenten wie vorstehend definiert sind.

Ein Aspekt der Erfindung sind Verbindungen der allgemeinen Formeln (1) oder (1A), worin
R³ 5-6 gliedriges Heteroaryl, gegebenenfalls substituiert mit einem oder mehreren, gleich oder verschiedenen R^{c} und/oder R^{b} bedeutet.

Ein Aspekt der Erfindung sind Verbindungen der allgemeinen Formeln (1) oder (1A), worin R³ unsubstituiertes Pyridyl, insbesondere Pyridin-3-yl bedeutet.

Ein Aspekt der Erfindung sind Verbindungen der allgemeinen Formeln (1) oder (1A), worin R¹ ausgewählt ist aus der Gruppe bestehend aus -NHC(O)R^{a}, -NHC(O)OR^{a} und -NHC(O)NR^{a}-R^{a}.
(A) Aspekte bezüglich R¹
   (A1) Ein Aspekt der Erfindung sind Verbindungen der allgemeinen Formel (1) oder (1A), worin R¹ -NHC(O)CH₂CH₃ bedeutet.
   (A2) Ein weiterer Aspekt der Erfindung sind Verbindungen der allgemeinen Formel (1) oder (1A), worin R¹ -NHC(O)OCH₃ oder -NHC(O)OCH₂CH₃ bedeutet.
   (A3) Ein Aspekt der Erfindung sind Verbindungen der allgemeinen Formel (1) oder (1A), worin R¹ -NHC(O)CH₃ bedeutet, und
      R² und R^{2'} jeweils unabhängig voneinander Wasserstoff oder ein Rest, ausgewählt aus der Gruppe bestehend aus C₃₋₆Alkyl, C₃₋₈Cycloalkyl, C₆₋₁₀Aryl, 5-10 gliedriges Heteroaryl und
      3-8 gliedriges Heterocycloalkyl, gegebenenfalls substituiert mit einem oder mehreren R^{a} und/oder R^{b}, oder
      R² und R^{2'} gemeinsam einen Heterocycloalkyl- oder Heteroarylring bilden, welcher gegebenenfalls ein oder mehrere weitere Heteroatome enthalten kann, ausgewählt aus der Gruppe bestehend aus N und S und gegebenenfalls mit einem oder mehreren R^{b} und/oder R^{d} substituiert sein kann, und
      worin
      jedes R^{b} ein geeigneter Rest und jeweils unabhängig voneinander ausgewählt aus der Gruppe bestehend aus =O, -OR^{c}, C₁₋₃Haloalkyloxy, =S, -SR^{c}, =NR^{c}, =NOR^{c}, -NR^{c}R^{c}, Halogen, -CF₃, -CN, -NC, -NO₂, -N₃, -S(O)R^{c}, -S(O)₂R^{c}, -S(O)₂OR^{c}, -S(O)NR^{c}R^{c}, - S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{d})OR^{c} -CN(R^{d})NR^{c}R^{c}, -OC(O)R^{c}, -OC(O)OR^{c}, -OC(O)NR^{c}R^{c}, -OCN(R^{d})NR^{c}R^{c}, -N(R^{d})C(O)R^{c}, -N(R^{d})C(S)R^{c}, -N(R^{d})S(O)₂R^{c}, -N(R^{d})C(O)OR^{c}, -N(R^{d})C(O)NR^{c}R^{c}, und -N(R^{d})CN(R^{d})NR^{c}R^{c}, und jedes R^{d} unabhängig voneinander C₁₋₆Alkyl, C₄₋₁₁Cycloalkylalkyl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl bedeuten.
(B) Aspekte bezüglich R² und R^{2'}
   (B1) Ein Aspekt der Erfindung sind Verbindungen der allgemeinen Formel (1) oder (1A), worin R² und R^{2'} jeweils unabhängig voneinander C₁₋₆Alkyl bedeuten.
   (B2) Ein weiterer Aspekt der Erfindung sind Verbindungen der allgemeinen Formel (1) oder (1A), worin R² und R^{2'} jeweils unabhängig voneinander Methyl oder Ethyl bedeuten.
   (B3) Ein weiterer Aspekt der Erfindung sind Verbindungen der allgemeinen Formel (1) oder (1A), worin R² C₃₋₈Cycloalkyl bedeutet.
   (B4) Ein weiterer Aspekt der Erfindung sind Verbindungen der allgemeinen Formel (1) oder (1A), worin R² Cyclopropyl bedeutet.
   (B5) Ein Aspekt der Erfindung sind Verbindungen der allgemeinen Formel (1) oder (1A), worin R² mit R^{2'} gemeinsam mit dem eingeschlossenen Stickstoffatom ein 3-8 gliedriges Heterocycloalkyl, welcher gegebenenfalls ein oder mehrere weitere Heteroatome enthalten kann, ausgewählt aus der Gruppe bestehend aus N, O und S bildet.
   (B6) Ein weiterer Aspekt der Erfindung sind Verbindungen der allgemeinen Formel (1) oder (1A), worin R² mit R^{2'} gemeinsam mit dem eingeschlossenen Stickstoffatom Azetidin, Pyrrolidin oder Piperidin bildet.
   (B7) Ein weiterer Aspekt der Erfindung sind Verbindungen der allgemeinen Formel (1) oder (1A), worin R² mit R^{2'} gemeinsam mit dem eingeschlossenen Stickstoffatom Thiazolidin, Thiomorpholin, Morpholin oder Piperazin bildet.
(C) Aspekte bezüglich R⁴
   (C1) Ein Aspekt der Erfindung sind Verbindungen der allgemeinen Formel (1) oder (1A), worin R⁴ Wasserstoff bedeutet.
   (C2) Ein weiterer Aspekt der Erfindung sind Verbindungen der allgemeinen Formel (1) oder (1A), worin R⁴ Fluor oder Chlor bedeutet.
(D) Aspekte bezüglich R⁵
   (D1) Ein Aspekt der Erfindung sind Verbindungen der allgemeinen Formel (1) oder (1A), worin R⁵ Wasserstoff bedeutet.
   (D2) Ein weiterer Aspekt der Erfindung sind Verbindungen der allgemeinen Formel (1) oder (1A), worin zwei R⁵ Cyclopropyl bilden.

Alle der oben genannten Aspekte (A1) bis (A4) für R¹, (B1) bis (B10) für R² und R^{2'} (C1) und (C2) für R⁴ und (D1) bis (D4) für R⁵ können beliebig miteinander kombiniert werden.

Die nachfolgende Tabelle stellt bevorzugte Kombinationen verschiedener Aspekte der erfindungsgemäßen Verbindungen der Formel (1) dar:

| Ausführungsform | R¹ | R² und R^{2'} | R⁴ | R⁵ |
|---|---|---|---|---|
| I-1 | A1 | B1 | C1 | D1 |
| I-2 | A1 | B2 | C1 | D1 |
| I-3 | A1 | B3 | C1 | D1 |
| I-4 | A1 | B4 | C1 | D1 |
| I-5 | A1 | B5 | C1 | D1 |
| I-6 | A1 | B6 | C1 | D1 |
| I-7 | A1 | B7 | C1 | D1 |
| I-8 | A1 | B8 | C1 | D1 |
| I-9 | A1 | B9 | C1 | D1 |
| I-10 | A1 | B1 | C2 | D1 |
| I-11 | A1 | B2 | C2 | D1 |
| I-12 | A1 | B3 | C2 | D1 |
| I-13 | A1 | B4 | C2 | D1 |
| I-14 | A1 | B5 | C2 | D1 |
| I-15 | A1 | B6 | C2 | D1 |
| I-16 | A1 | B7 | C2 | D1 |
| I-17 | A1 | B1 | C2 | D2 |
| I-18 | A1 | B2 | C2 | D2 |
| I-19 | A1 | B3 | C2 | D2 |
| I-20 | A1 | B4 | C2 | D2 |
| I-21 | A1 | B5 | C2 | D2 |
| I-22 | A1 | B6 | C2 | D2 |
| I-23 | A1 | B7 | C2 | D2 |
| I-24 | A1 | B1 | C1 | D2 |
| I-25 | A1 | B2 | C1 | D2 |
| I-26 | A1 | B3 | C1 | D2 |
| I-27 | A1 | B4 | C1 | D2 |
| I-28 | A1 | B5 | C1 | D2 |
| I-29 | A1 | B6 | C1 | D2 |
| I-30 | A1 | B7 | C1 | D2 |
| I-31 | A2 | B1 | C1 | D1 |
| I-32 | A2 | B2 | C1 | D1 |
| I-33 | A2 | B3 | C1 | D1 |
| I-34 | A2 | B4 | C1 | D1 |
| I-35 | A2 | B5 | C1 | D1 |
| I-36 | A2 | B6 | C1 | D1 |
| I-37 | A2 | B7 | C1 | D1 |
| I-38 | A2 | B8 | C1 | D1 |
| I-39 | A2 | B9 | C1 | D1 |
| I-40 | A2 | B1 | C2 | D1 |
| I-41 | A2 | B2 | C2 | D1 |
| I-42 | A2 | B3 | C2 | D1 |
| I-43 | A2 | B4 | C2 | D1 |
| I-44 | A2 | B5 | C2 | D1 |
| I-45 | A2 | B6 | C2 | D1 |
| I-46 | A2 | B7 | C2 | D1 |
| I-47 | A2 | B1 | C2 | D2 |
| I-48 | A2 | B2 | C2 | D2 |
| I-49 | A2 | B3 | C2 | D2 |
| I-50 | A2 | B4 | C2 | D2 |
| I-51 | A2 | B5 | C2 | D2 |
| I-52 | A2 | B6 | C2 | D2 |
| I-53 | A2 | B7 | C2 | D2 |
| I-54 | A2 | B1 | C1 | D2 |
| I-55 | A2 | B2 | C1 | D2 |
| I-56 | A2 | B3 | C1 | D2 |
| I-57 | A2 | B4 | C1 | D2 |
| I-58 | A2 | B5 | C1 | D2 |
| I-59 | A2 | B6 | C1 | D2 |
| I-60 | A2 | B7 | C1 | D2 |
| I-61 | A3 | B3 | C1 | D1 |
| I-62 | A3 | B4 | C1 | D1 |
| I-63 | A3 | B5 | C1 | D1 |
| I-64 | A3 | B6 | C1 | D1 |
| I-65 | A3 | B7 | C1 | D1 |
| I-66 | A3 | B8 | C1 | D1 |
| I-67 | A3 | B9 | C1 | D1 |
| I-68 | A3 | B1 | C2 | D1 |
| I-69 | A3 | B2 | C2 | D1 |
| I-70 | A3 | B3 | C2 | D1 |
| I-71 | A3 | B4 | C2 | D1 |
| I-72 | A3 | B5 | C2 | D1 |
| I-73 | A3 | B6 | C2 | D1 |
| I-74 | A3 | B/ | C2 | D1 |
| I-75 | A3 | B1 | C2 | D2 |
| I-76 | A3 | B2 | C2 | D2 |
| I-77 | A3 | B3 | C2 | D2 |
| I-78 | A3 | B4 | C2 | D2 |
| I-79 | A3 | B5 | C2 | D2 |
| I-80 | A3 | B6 | C2 | D2 |
| I-81 | A3 | B7 | C2 | D2 |
| I-82 | A3 | B1 | C1 | D2 |
| I-83 | A3 | B2 | C1 | D2 |
| I-84 | A3 | B3 | C1 | D2 |
| I-85 | A3 | B4 | C1 | D2 |
| I-86 | A3 | B5 | C1 | D2 |
| I-87 | A3 | B6 | C1 | D2 |
| I-88 | A3 | B7 | C1 | D2 |

Ein Aspekt der Erfindung sind Verbindungen der allgemeinen Formel (1) oder (1A), oder deren pharmakologisch verträglichen Salze, als Arzneimittel.

Ein Aspekt der Erfindung sind Verbindungen der allgemeinen Formel (1) oder (1A), oder deren pharmakologisch verträglichen Salze, zur Herstellung eines Arzneimittels mit antiproliferativer Wirkung.

Ein Aspekt der Erfindung ist eine pharmazeutische Zubereitungen, enthaltend als Wirkstoff eine oder mehrere Verbindungen der allgemeinen Formel (1) oder (1A), gemäß einem der Ansprüche 1 bis 5 oder deren pharmakologisch verträglichen Salze gegebenenfalls in Kombination mit üblichen Hilfs- und/oder Trägerstoffen.

Ein Aspekt der Erfindung ist dieVerwendung von Verbindungen der allgemeinen Formel (1) oder (1A), zur Herstellung eines Arzneimittels zur Behandlung und/oder Prävention von Krebs.

Ein Aspekt der Erfindung ist eine pharmazeutische Präparation umfassend eine Verbindung der allgemeinen Formel (1) oder (1A), und mindestens eine weitere zytostatische oder zytotoxische, von Formel (1) oder (1A) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze.

### Definitionen

Wie hierin verwendet treffen folgenden Definitionen zu, falls nicht anders beschrieben.

Unter Alkyl-Substitutenten sind jeweils gesättigte, ungesättigte, geradkettige oder verzweigte aliphatische Kohlenwasserstoffreste (Alkylrest) zu verstehen und umfasst sowohl gesättigte Alkylreste als auch ungesättigte Alkenyl- und Alkinylreste. Alkenyl-Substituenten sind jeweils geradkettige oder verzweigte, ungesättigte Alkylreste, die mindestens eine Doppelbindung aufweisen. Unter Alkinyl-Substituenten sind jeweils geradkettige oder verzweigte, ungesättigte Alkylreste, die mindestens eine Dreifachbindung aufweisen, zu verstehen.

Durch den Ausdruck Heteroalkyl werden Reste repräsentiert, die sich aus vorstehend definiertem Alkyl in seiner weitesten Bedeutung dadurch ableiten, dass in den Kohlenwasserstoffketten eine oder mehrere der Gruppen -CH₃ unabhängig voneinander durch die Gruppen -OH, -SH oder -NH₂, eine oder mehrere der Gruppen -CH₂- unabhängig voneinander durch die Gruppen -O-, -S- oder -NH- , eine oder mehrere der Gruppen 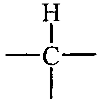 durch die Gruppe 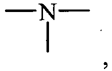 eine oder mehrere der Gruppen =CH- durch die Gruppe =N-, eine oder mehrere der Gruppen =CH₂ durch die Gruppe =NH oder eine oder mehrere der Gruppen ≡CH durch die Gruppe ≡N ersetzt werden, wobei insgesamt nur maximal drei Heteroatome in einem Heteroalkyl vorhanden sein können, zwischen zwei Sauerstoff- und zwischen zwei Schwefelatomen bzw. zwischen je einem Sauerstoff- und einem Schwefelatom mindestens ein Kohlenstoffatom vorliegen und der Rest insgesamt über chemische Stabilität verfügen muss.

Aus der indirekten Definition/Ableitung aus Alkyl ergibt sich unmittelbar, dass sich Heteroalkyl aus den Untergruppen gesättigte Kohlenwasserstoffketten mit Heteroatom(en), Heteroalkenyl und Heteroalkinyl zusammensetzt, wobei eine weitere Unterteilung in geradkettig (unverzweigt) und verzweigt vorgenommen werden kann. Sollte ein Heteroalkyl substituiert sein, so kann die Substitution unabhängig voneinander, jeweils ein- oder mehrfach, an allen wasserstofftragenden Sauerstoff-, Schwefel-, Stickstoff- und/oder Kohlenstoffatomen erfolgen. Heteroalkyl selbst kann als Substituent sowohl über ein Kohlenstoff- als auch über ein Heteroatom mit dem Molekül verknüpft sein.

Exemplarisch werden Vertreter im Folgenden aufgezählt:

Dimethylaminomethyl; Dimethylaminoethyl (1- Dimethylaminoethyl; 2-Dimethylaminoethyl); Dimethylaminopropyl (1-Dimethylaminopropyl, 2-Dimethylaminopropyl, 3-Dimethylaminopropyl); Diethylaminomethyl; Diethylaminoethyl (1-Diethylaminoethyl, 2-Diethylaminoethyl); Diethylaminopropyl (1-Diethylaminopropyl, 2- Diethylaminopropyl, 3-Diethylaminopropyl); Diisopropylaminoethyl (1-Diisopropylaminoethyl, 2-Diisopropylaminoethyl); Bis-2-methoxyethylamino; [2-(Dimethylamino-ethyl)-ethyl-amino]-methyl; 3-[2-(Dimethylamino-ethyl)-ethyl-amino]-propyl; Hydroxymethyl; 2-Hydroxyethyl; 3-Hydroxypropyl; Methoxy; Ethoxy; Propoxy; Methoxymethyl; 2-Methoxyethyl etc.

Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise -CF₃, -CHF₂, -CH₂F, -CF₂CF₃,-CHFCF₃, -CH₂CF₃, -CF₂CH₃, -CHFCH₃, -CF₂CF₂CF₃, -CF₂CH₂CH₃, -CF=CF₂, -CC1=CH₂, -CBrCH₂, -Cl=CH₂, -C≡C-CF₃, -CHFCH₂CH₃ und -CHFCH₂CF₃.

Halogen bezieht sich auf Fluor-, Chlor-, Brom- und/oder Iodatome.

Unter Cycloalkyl ist ein mono- oder bizyklischer Ring zu verstehen, wobei das Ringsystem ein gesättigter Ring aber auch ein ungesättigter, nichtaromatischer Ring sein kann, welcher gegebenenfalls auch Doppelbindungen enthalten kann, wie zum Beispiel Cyclopropyl, Cyclopropenyl, Cyclobutyl, Cyclobutenyl, Cyclopentyl, Cyclopentenyl, Cyclohexyl, Cyclohexenyl, Norbornyl und Norbornenyl.

Cycloalkylalkyl umfasst eine nicht-zyklische Alkylgruppe, in der ein an einem Kohlenstoffatom gebundenes Wasserstoffatom, üblicherweise an einem terminalen C-Atom, durch eine Cycloalkylgruppe ersetzt ist.

Aryl bezieht sich auf monozyklische oder bizyklische aromatische Ringe mit 6 - 10 Kohlenstoffatomen wie beispielsweise Phenyl und Naphthyl.

Arylalkyl umfasst eine nicht-zyklische Alkylgruppe, in der ein an einem Kohlenstoffatom gebundenes Wasserstoffatom, üblicherweise an einem terminalen C-Atom, durch eine Arylgruppe ersetzt ist.

Unter Heteroaryl sind mono- oder bizyklische aromatische Ringe zu verstehen, welche anstelle eines oder mehrere Kohlenstoffatome ein oder mehrere, gleich oder verschiedene Heteroatome enthalten, wie z.B. Stickstoff-, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, Benzofuryl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, Oxazolopyridyl, Imidazopyridyl, Naphthyridinyl, Indolinyl, Isochromanyl, Chromanyl, Tetrahydroisochinolinyl, Isoindolinyl, Isobenzotetrahydrofuryl, Isobenzotetrahydrothienyl, Isobenzothienyl, Benzoxazolyl, Pyridopyridyl, Benzotetrahydrofuryl, Benzotetrahydrothienyl, Purinyl, Benzodioxolyl, Triazinyl, Phenoxazinyl, Phenothiazinyl, Pteridinyl, Benzothiazolyl, Imidazopyridyl, Imidazothiazolyl, Dihydrobenzisoxazinyl, Benzisoxazinyl, Benzoxazinyl, Dihydrobenzisothiazinyl, Benzopyranyl, Benzothiopyranyl, Coumarinyl, Isocoumarinyl, Chromonyl, Chromanonyl, Pyridyl-*N*-oxid Tetrahydroquinolinyl, Dihydroquinolinyl, Dihydroquinolinonyl, Dihydroisoquinolinonyl, Dihydrocoumarinyl, Dihydroisocoumarinyl, Isoindolinonyl, Benzodioxanyl, Benzoxazolinonyl, Pyrrolyl-*N*-oxid, Pyrimidinyl-*N*-oxid, Pyridazinyl-*N*-oxid, Pyrazinyl-*N-*oxid, Quinolinyl-*N*-oxid, Indolyl-*N*-oxid, Indolinyl-*N*-oxid, Isoquinolyl-*N*-oxid, Quinazolinyl-*N*-oxid, Quinoxalinyl-*N*-oxid, Phthalazinyl-*N*-oxid, Imidazolyl-*N*-oxid, Isoxazolyl-*N*-oxid, Oxazolyl-*N*-oxid, Thiazolyl-*N*-oxid, Indolizinyl-*N*-oxid, Indazolyl-*N-*oxid, Benzothiazolyl-*N*-oxid, Benzimidazolyl-*N*-oxid, Pyrrolyl-*N*-oxid, Oxadiazolyl-*N-*oxid, Thiadiazolyl-*N*-oxid, Triazolyl-*N*-oxid, Tetrazolyl-*N*-oxid, Benzothiopyranyl-*S*-oxid und Benzothiopyranyl-*S,S*-dioxid.

Heteroarylalkyl umfasst eine nicht-zyklische Alkylgruppe, in der ein an einem Kohlenstoffatom gebundenes Wasserstoffatom, üblicherweise an einem terminalen C-Atom, durch eine Heteroarylgruppe ersetzt ist.

Heterocycloalkyl bezieht sich auf 3 - 12 Kohlenstoffatome umfassende gesättigte oder ungesättigte, nicht aromatische mono-, bizyklische oder überbrückte bizyklische Ringe, welche anstelle eines oder mehrere Kohlenstoffatome Heteroatome, wie Stickstoff, Sauerstoff oder Schwefel, tragen. Beispiele für solche Heterocyloalkylreste sind Tetrahydrofuryl, Pyrrolidinyl, Pyrrolinyl, Imidazolidinyl, Imidazolinyl, Pyrazolidinyl, Pyrazolinyl, Piperidinyl, Piperazinyl, Indolinyl, Isoindolinyl, Morpholinyl, Thiomorpholinyl, Homomorpholinyl, Homopiperidinyl, Homopiperazinyl, Homothiomorpholinyl, Thiomorpholinyl-S-oxid, Thiomorpholinyl₋*S,S*-dioxid, Tetrahydropyranyl, Tetrahydrothienyl, Homothiomorpholinyl-S,S-Dioxid, Oxazolidinonyl, Dihydropyrazolyl, Dihydropyrrolyl, Dihydropyrazinyl, Dihydropyridyl, Dihydropyrimidinyl, Dihydrofuryl, Dihydropyranyl, Tetrahydrothienyl-S-oxid, Tetrahydrothienyl-S,S-dioxid, Homothiomorpholinyl-S-oxid, 2-Oxa-5-azabicyclo[2.2.1]heptan, 8-Oxa-3-aza-bicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diazabicyclo[4.2.1]nonan und 2,6-Diaza-bicyclo[3.2.2]nonan.

Heterocycloalkylalkyl bezieht sich auf eine nicht-zyklische Alkylgruppe, in der ein an einem Kohlenstoffatom gebundenes Wasserstoffatom, üblicherweise an einem terminalen C-Atom, durch eine Heterocycloalkylgruppe ersetzt ist.

Die nachfolgenden Beispiele illustrieren die vorliegende Erfindung ohne sie jedoch in ihrem Umfang einzuschränken.

### Analytische Methoden

### Methode AM1:

HPLC: Agilent 1100 Series; MS: 1100 Series LC/MSD (API-ES (+/- 3000V, Quadrupol, G1946D); Mode: Scan pos 100-1000, neg 100-1000

| | |
|---|---|
| Säule: | Waters; Part No.186000594; XTerra MS C18 2.5µm; 2.1x50mm column |
| Lösungsmittel: | A: H₂O entsalzt mit 0.1% Ameisensäurezusatz |
| | B: Acetonitril HPLC grade mit 0.1% Ameisensäurezusatz |
| Detektion: | peakwide >0.1 min (2 s); 190 - 450 nm |
| | UV 254 nm (bandwide 8, reference off) |
| | UV 230 nm (bandwide 8, reference off) |
| Injektion: | 1 µL standard injection |
| Fluss: | 0.6 mL/min |
| Säulentemperatur: | 35 C° |
| Pumpengradient: | 0.0 - 0.5 min 5% B |
| | 0.5 - 1.5 min 5% -> 50% B |
| | 1.5 - 4.0 min 50%->95%B |
| | 4.0 - 6.0 min 95% B |
| | 6.0 - 6.5min 95%->5%B |
| | 1.5 min post run 5% B |

### Methode AM2

HPLC: Agilent Series 1100 (G1379A/G1310A umgerüstet auf G1311A/G1313A/G1316A/G1948D/G1315B/G1946D) Mode: Scan pos 100-1000, neg 100-1000

| | |
|---|---|
| Säule: | Agilent Zorbax SB-C8, 2.1x50 mm, 3.5 µm |
| Lösungsmittel: | A: H₂O entsalzt mit 0. 1 % Ameisensäurezusatz |
| | B: Acetonitril HPLC grade mit 0. 1 % Ameisensäurezusatz |
| Detektion: | peakwide >0.1 min (2s); 190-450nm |
| | UV 254 nm (bandwide 8, reference off) |
| | UV 230 nm (bandwide 8, reference off) |
| Injektion: | 2.5 µL standard injection |
| Fluss: | 0. 6 mL/min |
| Säulentemperatur: | 35 C° |
| Pumpengradient: | 0 - 3.0 min 10% -> 90% B |
| | 3.0 - 4.0 min 90% B |
| | 4.0 - 5.0 min 90%-> 10% B |

### Methode AM3

HPLC: Agilent Series 1100 (G1312A/G1315A/G1316A/G1367A) Agilent MSD SL ESI Mode: Scan pos 150-750

| | |
|---|---|
| Säule: | Agilent Zorbax SB-C8, 2.1x50 mm, 3.5 µm |
| Lösungsmittel: | A: H₂O entsalzt mit 0.1 % Ameisensäurezusatz |
| | B: Acetonitril HPLC grade mit 0.1% Ameisensäurezusatz |
| Detektion: | peakwidth >0.01 min (0.2 s); 190 - 450nm |
| | UV 254 nm (bandwide 16, reference off) |
| | UV 230 nm (bandwide 8, reference off) |
| | UV 214 nm (bandwide 8, reference off) |
| Injektion: | 3.0 µL overlap injection |
| Fluss: | 1.1 mL/min |
| Säulentemperatur: | 45 °C |
| Pumpengradient: | 0 - 1.75 min 15% -> 95% B |
| | 1.75 - 1.90 min 95% B |
| | 1.90 - 1.92 min 950%-> 15% B |

### Methode AM4

| | | |
|---|---|---|
| HPLC: | Agilent 1100 Series | |
| MS: | Agilent LC/MSD SL | (LCMS1: 1100 series LC/MSD) |
| Säule: | Waters, Xterra M S C18, 2.5 µm, 2.1x30 mm, Part.No.186000592 | |
| Lösungsmittel | A: H₂O entsalzt mit 0.1 % Ameisensäurezusatz | |
| | B: Acetonitril HPLC grade mit 0.1% Ameisensäurezusatz | |

| | | |
|---|---|---|
| Detektion: | MS: | Positive and negative |
| | Mass range: | 120 - 900 m/z |
| | Fragmentor: | 120 |
| | Gain EMV: | 1 |
| | Threshold: | 150 |
| | Stepsize: | 0.25 |
| | UV: | 254 nm |
| | Bandwide: | 1 (LCMS1: 2) |
| | Reference: | off |
| | Spectrum: | |
| | Range: | 250 - 400 nm |
| | Range step: | 1.00 nm |
| | Threshold: | 4.00 mAU |
| | Peakwidth: | <0.01 min (LCMS1: >0.05 min) |
| | Slit: | 1 nm (LCMS1: 2 nm) |
| Injektion: | 5 µL | |
| Fluß: | 1.10 mL/min | |
| Säulentemperatur: | 40 °C | |
| Gradient: | 0.00 min | 5%B |
| | 0.00 - 2.50 min | 5%->95%B |
| | 2.50 - 2.80 min | 95% B |
| | 2.81 - 3.10 min | 95%->5%B |

### Methode AM5

| | | | |
|---|---|---|---|
| HPLC: | Agilent 1100 Series | | |
| MS: | Agilent LC/MSD SL | (LCMS1 : 1100 series LC/MSD) | |
| Column: | Phenomenex, Synergi Polar RP 80A, 4 µm, 2x30 mm, Part.No.00A-4336- BO | | |
| | | | |
| Solvent: | A: | H₂O (Millipore purified purest water) with 0.1% Ameisensäurezusatz | |
| | B: | Acetonitril (HPLC grade) | |
| Detection: | MS: | Positive and negative | |
| | Mass range: | 120 - 900 m/z | |
| | Fragmentor: | 120 | |
| Gain EMV: | 1 | | |
| Threshold: | 150 | | |
| Stepsize: | 0.25 | | |
| UV: | 254 nm | | |
| Bandwide: | 1 | | (LCMS1: 2) |
| Reference: | off | | |
| Spectrum: | | | |
| | Range: | 250 - 400 nm | |
| | Range step: | 1.00 nm | |
| | Threshold: | 4.00 mAU | |
| | Peakwidth: | < 0.01 min | (LCMS1 : >0.05 min) |
| | Slit: | 1 nm | (LCMS1: 2 nm) |
| Injection: | Inj. Vol.: | 5 µL | |
| Inj. mode: | Needle wash | | |
| Separation: | Flow: | 1.10 mL/min | |
| | Column temp.: | 40 °C | |
| | Gradient: | 0.00 min | 5 % solvent B |
| | | 0.00 - 2.50 min | 5 % -> 95 % solvent B |
| | | 2.50 - 2.80 min | 95 % solvent B |
| | | 2.81 - 3.10 min | 95 % -> 5 % solvent B |

### Methode AM6

| | | |
|---|---|---|
| HPLC: | Waters Alliance 2695 | |
| Säule: | Waters, Xterra MS C18, 2.5µm, 4.6x30 mm, Part.No.186000600 | |
| Lösungsmittel | A: H₂O entsalzt mit 0.1% Ameisensäurezusatz | |
| | B: Acetonitril HPLC grade mit 0.08 % Ameisensäurezusatz | |
| Fluß: | 1 mL/min | |
| Säulentemperatur: | 25 °C | |
| Gradient: | 0.00 min | 5 % B |
| | 0.00 - 3.10 min | 5%->98%B |
| | 3.10 - 4.50 min | 98 % B |
| | 4.50 - 5.00 min | 98 % -> 5 % B |

### Verwendete Abkürzungen

| | |
|---|---|
| d | Tag |
| DC | Dünnschichtchromatographie |
| DMF | *N,N*-Dimethylformamid |
| DMSO | Dimethylsulfoxid |
| h | Stunde |
| HPLC | Hochdurchsatzflüssigchromatographie |
| M | Molar |
| min | Minute |
| mL | Milliliter |
| MS | Massenspektrometrie |
| N | Normal |
| NMR | Kernresonanzspektroskopie |
| ppm | part per million |
| Rf | Retentionsfaktor |
| RP | Reversed Phase |
| RT | Raumtemperatur |
| Rt | Retentionszeit |
| Smp | Schmelzpunkt |
| *tert* | tertiär |
| THF | Tetrahydrofuran |

### Synthese der Reagenzien

### H-1)(2-Chlor-4-morpholin-4-ylmethyl-phenyl)-hydrazin Dihydrochlorid

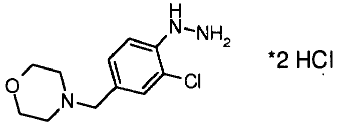

### H-1a) 4-Brom-3-chlorbenzylbromid

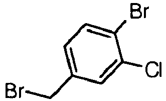

Ein Suspension von N-Bromsuccinimid (16.4 g, 87.4 mmol), 4-Brom-chlortoluol (18.2 g, 87.4 mmol) und α,α'-Azoisobutyronitril (0.67 g, 4 mmol) in 90 mL Dichlormethan wird in der Mikrowelle 1 min bei 100 °C erhitzt und anschließend über Nacht bei -20 °C gekühlt. Der entstandene weiße Niederschlag wird filtriert und das Filtrat wird eingeengt. Der Rückstand wird mit 100 mL Diethylether verrührt, filtriert, eingeengt und nochmals filtriert. Ausbeute: 24.1 g

### H-1b) 1-Brom-2-chlor-4-morpholin-4-ylmethyl-benzol

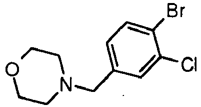

Zu einer Lösung von Morpholin (23.1 mL, 262 mmol) in 250 mL Tetrahydrofuran wird bei 0 °C sehr langsam eine Lösung von H-1a (24.1 g, 84.7 mmol) zugetropft. Die Reaktionsmischung wird über Nacht bei RT gerührt, filtriert und eingeengt. Der Rückstand wird nacheinander mit 250 mL Dichlormethan, 100 mL Wasser und anschließend mit 200 mL 1 M, wässeriger Salzsäure versetzt. Nach Kühlen im Eisbad wird der ausgefallene Niederschlag filtriert und zweimal mit Eiswasser sowie anschließend mit Dichlormethan gewaschen. Ausbeute: 12.9 g

Anschließend wird diese Zwischenverbindung mit 100 mL Wasser versetzt und mit wässrige Kaliumcarbonatlösung auf pH 9 gestellt. Die wässrige Phase wird dreimal mit je 150 mL Ethylacetat ausgeschüttelt. Die vereinigten organischen Phasen werden über Magnesiumsulfat getrocknet, filtriert und eingeengt. Ausbeute 11.2 g

### H-1c) N-(2-Chlor-4-morpholin-4-ylmethyl-phenyl)-benzophenon-hydrazon

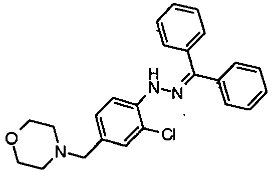

Unter Argonatmosphäre wird zu einer Mischung von Benzophenonhydrazon (7.72 g, 38.5 mmol), Natrium-*tert*-butoxid (5.73 g, 57.8 mmol), Palladium(II)acetat (0.13 g, 0.58 mmol) und rac-BINAP (0.49 g, 0.77 mmol) eine Lösung von H-1b (11.2 g, 38.5 mmol) in 500 mL Toluol zugegeben. Die Reaktionsmischung wird 3 h bei Rückfluss gerührt und anschließend mit 10 g Celite versetzt, über ein Celite-Bett filtriert und eingeengt. Der Rückstand wird mit 50 mL 1-Propanol versetzt und über Nacht stehen gelassen. Der ausgefallene Niederschlag wird filtriert. Ausbeute: 10.9 g.

Zu H-1c (10.8 g, 26.6 mmol) wird 30 mL 1-Propanol und 30 mL 37% Salzsäure zugegeben und die Reaktionsmischung wird 1.5 min bei 130 °C in der Mikrowelle erhitzt. Das gewünschte Produkt kristallisiert nach einiger Zeit aus. Ausbeute: 6.69 g.

### H-2) (4-[1-N,N-Dimethylamino-cyclopropyl-1-yl]-phenyl)-hydrazin Dihydrochlorid

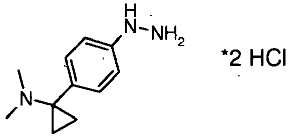

### H-2a) 1-(4-Bromphenyl)-1-cyclopropylcarbonsäure

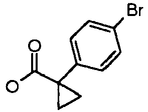

Zu einer Lösung von 1-Phenyl-1-cyclopropylcarbonsäure (5 g, 30 mmol) und Natriumacetat (2.7 g, 33 mmol) in 30 mL Eisessig wird bei 5 °C Brom (1.5 mL, 30 mmol) zugetropft. Anschließend wird auf 50 °C erwärmt und 2 Tage gerührt. Die Reaktionsmischung wird mit 100 mL Wasser verdünnt, und der ausgefallene Niederschlag abfiltriert sowie mit 50% Essigsäure gewasche. Ausbeute: 5.7 g.

### H-2b) 1-(4-Bromphenyl)-1-cyclopropylcarbamidsäure-tert-butylester

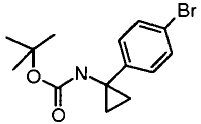

Unter Argonatmosphäre wird ein Suspension von H-2a (5.7 g, 24 mmol), *N*-Ethyldiisopropylamin (5.3 mL, 31 mmol), *tert*-Butanol (55 mL, 0.59 mol), Diphenylphosphorylazid (6.1 mL, 28 mmol) und aktiviertes Molsieb (4 Å) in 120 mL Toluol 5 h unter Rückfluss erhitzt. Das Reaktionsgemisch wird eingeengt und der Rückstand mit 100 mL Ethylacetat versetzt und mit je 50 mL 5%iger Zitronensäurelösung, gesättigter Natriumhydrogencarbonatlösung und gesättigter Natriumchloridlösung ausgeschüttelt. Die organische Phase wird über Magnesiumsulfat getrocknet und eingeengt. Ausbeute 7.7 g.

### H-2c) N-Methyl-1-(4-bromphenyl)-1-cyclopropylcarbamidsäure-tert-butylester

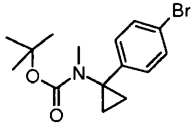

Zu einer Lösung von H-2b (32 g, 0.1 mol) in 300 mL DMF wird bei RT Natriumhydrid (60% in Mineralöl, 6.2 g, 0.15 mol) zugegeben. Nach 10 min wird auf 40° erwärmt und 15 min später wird Methyliodid (14 mL, 0.23 mol) zugegeben und noch 1 h bei 40 °C gerührt. Das Reaktionsgemisch wird auf 1.2 L Eiswasser gegeben, 15 min gerührt und anschließend dreimal mit je 500 mL Ethylacetat ausgeschüttelt. Die vereinigten organischen Phasen werden über Magnesiumsulfat getrocknet und eingeengt. Ausbeute: 38 g (w = 0.85)

### H-2d) N-Methyl-1-(4-bromphenyl)-1-cyclopropylamin Hydrochlorid

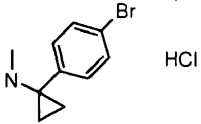

Eine Lösung von H-2c (34 g, 0.1 mol) in 260 mL Salzsäure (4 M in Dioxan) wird 1 h bei RT gerührt und anschließend eingeengt. Ausbeute: 31 g (w = 0.85)

### H-2e) N,N-Dimethyl-1-(4-bromphenyl)-1-cyclopropylamin Hydrochlorid

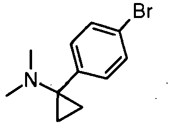

Zu einer Lösung von H-2d (30 g, 0.1 mol) in 400 mL Dichlorethan/Methanol (1:1) wird langsam 35%iger Formaldehyd in Wasser (40 mL, 0.5 mol) zugegeben und 30 min heftig bei RT gerührt. Anschließend wird Eisessig (8.6 mL, 0.15 mol) und Natriumtrisacetoxyborhydrid (32 g, 0.15 mol) portionsweise zugegeben und über Nacht bei RT gerührt. Das Reaktionsgemisch wird mit 500 mL gesättigter Natriumhydrogencarbonatlösung versetzt und 30 min nachgerührt. Die Phasen werden getrennt und die wässerige Phase wird mit 500 mL Dichlormethan extrahiert. Die vereinigten organischen Phasen werden getrocknet und eingeengt, und der Rückstand wird chromatographisch an Kieselgel mit Cyclohexan/Ethylacetat gereinigt. Ausbeute: 10.2 g.

### H-2f) N-([1-N,N-Dimethylamino-1cyclopropyl-1-yl]-phenyl)-benzophenonhydrazon

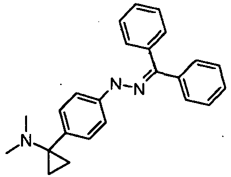

Analog zu Darstellung von H-1c wird ausgehend von H-2e (10 g, 42 mmol), Benzophenonhydrazon (8.3 g, 42 mmol), Natrium-*tert*-butoxid (6.1 g, 64 mmol), Palladium(II)acetat (0.19 g, 0.85 mmol) und *rac*-BINAP (1.1 g, 1.7 mmol) die gewünschte Verbindung erhalten. Ausbeute: 9.1 g.

Eine Lösung von H-2f (8.9 g, 25 mmol) in 30 mL 1-Propanol und 31 mL 37%iger Salzsäure wird 1 h unter Rückfluss gerührt. Das Reaktionsgemisch wird eingeengt, nochmals in 1-Propanol aufgenommen und wieder eingeengt. Der Rückstand wird über Nacht in 150 mL Acetonitril gerührt, und der entstandene Niederschlag abfiltriert. Ausbeute: 4.3 g.

### H-3) [4-(1-Pyrrolidin-1-yl-cyclopropyl)-phenyl]-hydrazin Bis-Trifluoracetat

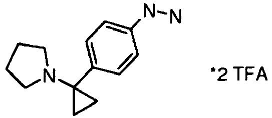

### H-3a) 1-(4-Bromphenyl)-1-cyclopropylamin Hydrochlorid

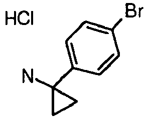

Zu einer Lösung von H-2b (19.5 g, 37.4 mmol) in 50 mL Dioxan wird 47 mL Salzsäure (4 M in Dioxan) zugegeben und über Nacht bei RT gerührt. Der entstandene Niederschlag wird abfiltriert, das Filtrat auf ca. die Hälfte eingeengt und nochmals filtriert. Die Filterkuchen werden vereint und getrocknet. Ausbeute: 10.1 g (w = 0.90)

### H-3b) 4-Brom-(1-Pyrrolidin-1-yl-cyclopropyl-1-yl)-benzol

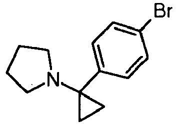

Zu einer Suspension von H-3a (0.5 g, 1.7 mmol), Kaliumcarbonat (0.73 g, 5.2 mmol) und Kaliumiodid (3 mg, 0.02 mmol) in 10 mL Acetonitril wird 1,4-Dibrombutan (0.19 mL, 1.7 mmol) zugegeben und anschließend 24 h unter Rückfluss erhitzt. Das Reaktionsgemisch wird abfiltriert, eingeengt und der Rückstand chromatographisch mittels RP-HPLC gereinigt. Ausbeute: 0.19 g.

### H-3c) N,N'-[4-(1-Pyrrolidin-1-yl-cyclopropyl)-phenyl]-hydrazindicarbonsäure-di-tert-butylester

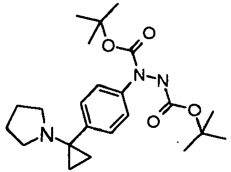

Zu einer Lösung von *n*-Butyllithium (0.84 mL, 2.5 M in Hexan) in 5 mL trocknem THF wird bei -78 °C unter Argonatmosphäre eine Lösung von H-3b (0.19 g, 0.7 mmol) in 3 mL trockenem THF zugegeben. Danach wird eine Lösung von Di-*tert*-butyl-azodicarboxylat (0.19 g, 0.84 mmol) in 4 mL trocknes THF zugegeben. Nach 5 min wird das Kältebad entfernt und nach weiteren 20 min wird das Reaktionsgemisch mit 10 mL Wasser und 20 mL Ethylacetat versetzt. Die Phasen werden getrennt und die wässerige Phase wird mit 10 mL Ethylacetat extrahiert. Die vereinigten organischen Phasen werden über Magnesiumsulfat getrocknet und eingeengt. Ausbeute: 0.31 g (w = 0.9)

Zu einer Lösung von H-3c (0.29 g, 0.7 mmol) in 4 mL Dichlormethan wird Trifluoressigsäure (0.1 mL, 2 mmol) zugegeben und 1 h bei RT gerührt. Anschließend wird das Reaktionsgemisch eingeengt. Ausbeute: 0.33 g (w = 0.9)

### H-4) (4-[1-Morpholin-1-yl-cyclopropyl-1-yl]-phenyl)-hydrazin Bis-Trifluoracetat

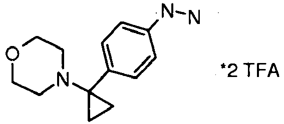

### H-4a) 4-Brom-(1-Morpholin-1-yl-cyclopropyl-1-yl)-benzol

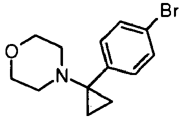

Analog zur Darstellung von H-3b wird ausgehend von H-3a (0.5 g, 1.7 mmol), Kaliumcarbonat (0.73 g, 5.2 mmol), Kaliumiodid (3 mg, 0.02 mmol) und Bis(2-bromethyl)ether die gewünschte Verbindung erhalten. Ausbeute: 0.32 g.

### H-4b) N,N'-[4-(1-Morpholin-4-yl-cyclopropyl)-phenyl]-hydrazindicarbonsäure-di-tert-butylester

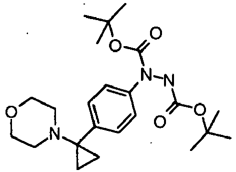

Analog zur Darstellung von H-3c wird ausgehend von H-4a (0.22 g, 0.76 mmol), *n*-Butyllithium (0.92 mL, 2.5 M in Hexan) und Di-*tert*-butylazodicarboxylat (0.21 g, 0.92 mmol) die gewünschte Verbindung erhalten. Ausbeute: 0.34 g.

Analog zur Darstellung von H-3 wird ausgehend von H-4b (0.29 g, 0.7 mmol) und Trifluoressigsäure (0.1 mL, 2 mmol) die gewünschte Verbindung erhalten. Ausbeute: 0.36 g (w = 0.9)

### H-5) (4-Imidazol-1-ylmethyl-phenyl)-hydrazin

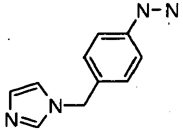

### H-5a) 1-(4-Nitro-benzyl)-1H-imidazol

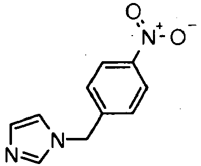

Zu einer Lösung von 4-Nitrobenzylbromid (5 g, 23 mmol) und Imidazol (1.6 g, 23 mmol) wird Kaliumcarbonat (3.1 g, 23 mmol) zugegeben und das Reaktionsgemisch bei RT gerührt. Nach vollständigem Umsatz laut HPLC-MS wird die Suspension in 800 mL Wasser gegossen und dreimal mit Ethylacetat extrahiert. Die vereinigten organischen Phasen werden mit gesättigte Natriumchloridlösung gewaschen, über Natriumsulfat getrocknet und eingeengt. Der Rückstand wird chromatographisch an Kieselgel mit Dichlormethan/Methanol gereinigt. Ausbeute: 2.1 g.

### H-5b) 4-Imidazol-1-ylmethyl-phenylamin

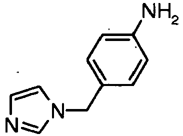

Eine Lösung von H-5a (2.1 g, 10 mmol) in 10 mL Ethanol, 10 mL Ethylacetat und 1.3 mL 1 M Salzsäure wird in einem Hydrierreaktor mit Palladium (10% auf Aktivkohle, 0.27 g) versetzt und über Nacht bei RT und 3.4 bar Wasserstoffdruck gerührt. Das Reaktionsgemisch wird filtriert, eingeengt und dreimal mit Ethanol kodestilliert. Ausbeute: 1.8 g. Zu einer Lösung von H-5b (1.8 g, 10 mmol) in 10 mL 37% Salzsäure wird bei -10 °C eine Lösung von Natriumnitrit (0.77 g, 10 mmol) in 5 mL Wasser zugegeben. Nach 20 min Rühren bei 0 °C wird wieder bei -10 °C eine Lösung von Zinn(II)chlorid Dihydrat (8.9 g, 39 mmol) in 10 mL 37% Salzsäure zugegeben. Das Reaktionsgemisch wird auf RT erwärmt, mit 10 N Natronlauge basisch gestellt und mit Ethylacetat extrahiert. Die wässrige Phase wird filtriert und mit Dichlormethan extrahiert. Die vereinigten organischen Phasen werden getrocknet und eingeengt. Ausbeute: 0.32 g.

### H-6) 2-Fluor-4-hydrazino-benzonitril Hydrochlorid

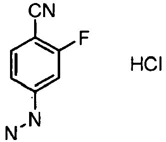

### H-6a) 4-Amino-2-fluor-benzonitril

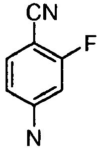

Eine Lösung von 2-Fluor-4-nitrobenzonitril (5 g, 30 mmol) in 300 mL Methanol wird in einem Hydrierreaktor mit Palladium (5% auf Aktivkohle, 400 mg) 2 h bei RT und 2 bar Wasserstoffdruck gerührt. Das Reaktionsgemisch wird filtriert und eingeengt. Ausbeute: 4 g.

Analog zur Darstellung von H-5 wird ausgehend von H-6a (4 g, 29 mmol), Natriumnitrit (3.2 g, 46 mmol) und Zinn(II)chlorid Dihydrat (26 g, 0.12 mol) die gewünschte Verbindung nach Versetzen mit 10 mL einer 2 M-Lösung von HCl in Dioxan als Hydrochlorid erhalten. Ausbeute: 4.3 g (w = 0.7)

### H-7) 4-Fluor-3-hydrazino-benzonitril

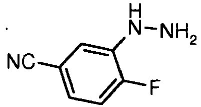

4-Fluor-3-nitrobenzonitril (5 g, 30 mmol) wird zunächst einer katalytischen Hydrierung mit 5%-Palladium auf Aktivkohle (0.4 g) in 300 mL Methanol unterzogen (2 h bei 2 bar Wasserstoffdruck), wobei das gebildete 3-Amino-4-fluorbenzonitril nach Filtration und Evaporation des Lösungsmittels als Feststoff anfällt. Ausbeute: 4.1 g.

Analog zur Darstellung von H-5 wird ausgehend von 3-Amino-4-fluorbenzonitril (4.1 g, 30 mmol), Natriumnitrit (3.1 g, 45 mmol), Zinn(II)chlorid Dihydrat (27 g, 119 mmol) in insgesamt 130 mL Salzsäure (w = 0.32) und 40 mL Wasser das gewünschte Produkt H-7 erhalten. Ausbeute: 4.1 g

### Z-1) N-[7-Oxo-6-(pyridin-3-carbonyl)-4,5,6,7-tetrahydro-benzthiazol-2-yl]-acetamid

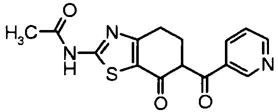

Die Synthese von Z-1 ist in DE102004048877 beschrieben.

### Z-2) [7-Oxo-6-(pyridin-3-carbonyl)-4,5,6,7-tetrahydro-benzothiazol-2-yl]-carbamidsäuremethylester

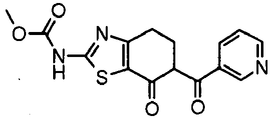

### Z-2a) [7-Oxo-4,5,6,7-tetrahydro-benzthiazol-2-yl]-carbamidsäuremethylester

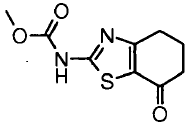

Zu einer Suspension von 2-Amino-5,6-dihydro-4-*H*-benzthiazol-7-on (30 g, 0.18 mol) in 500 mL trockenem THF wird eine Lösung von Methylchlorofornziat (15 mL, 0.2 mol) in 15 mL trockenem THF zugegeben und anschließend über Nacht unter Rückfluss gerührt. Das Reaktionsgemisch wird filtriert, das Filtrat eingeengt, und der feste Rückstand mit Diethylether verrührt, erneut filtriert und getrocknet. Ausbeute: 20 g.

Zu einer Lösung von Z-2a (15 g, 66 mmol) in 1.2 L trockenem THF wird bei -10 °C unter Argonatmosphäre 200 mL LiHMDS (1M in THF) zugetropft und 4 h gerührt. Anschließend wird zur Suspension eine Lösung von 3-Pyridylcarbonyl-*N*-imidazolid (23 g, 0.13 mol) in 85 mL trockenem THF zugegeben. Nach 1 h wird das Kältebad entfernt und das Reaktionsgemisch über Nacht bei RT gerührt. Das Lösungsmittel wird abdekantiert und der Rückstand mit Dichlormethan und gesättigter Natriumhydrogencarbonatlösung versetzt. Die Phasen werden getrennt und die wässrige Phase wird mit Dichlormethan extrahiert. Die vereinigten organischen Phasen werden über Magnesiumsulfat getrocknet, eingeengt, und der Rückstand wird mit 200 mL Diethylether verrührt. Der Niederschlag wird filtriert, in 200 mL Acetonitril 1 h mit Ultraschall behandelt und abfiltriert. Ausbeute: 9.3 g.

### Z-3) N-[1-(2-Chlor-4-formyl-phenyl)-3-pyridin-3-yl-4,5-dihydro-1H-pyrazolo[4,3-g]benzthiazol-7-yl]-acetamid

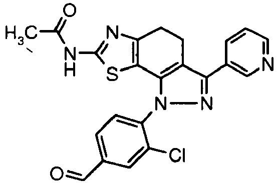

In einem Hydrierreaktor unter Argonatmosphäre wird bei 10 °C zu einer Suspension von Raney-Nickel (8 g, 93 mmol) in 75 mL Dichlormethan und 75 mL Methanol Triethylammonium Hypophsophithydrat (2.2 mL, 12 mmol) zugegeben. Nach dem Abklingen der Gasentwicklung wird *N*-[1-(2-Chlor-4-cyanophenyl)-3-pyridin-3-yl-4,5-dihydro-1*H*-pyrazolo[4,3-*g*]benzthiazol-7-yl]-acetamid (PCT/EP05055021) (2.7 g, 5.6 mmol) zugegeben und 3 h bei 55 °C gerührt. Das Reaktionsgemisch wird filtriert und das Filtrat eingeengt. Der Rückstand wird an Kieselgel mit Dichlormethan/Methanol gereinigt. Ausbeute: 1.1 g.

### Z-4) N-[1-(3-Dimethylamino-4-formyl-phenyl)-3-pyridin-3-yl-4,5-dihydro-1H-pyrazolo[4,3-g]benzthiazol-7-yl]-acetamid

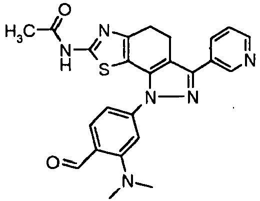

### Z-4a) N-[1-(3-Chlor-4-cyanophenyl)-3-pyridin-3-yl-4,5-dihydro-1H-pyrazolo[4,3-g]benzthiazol-7-yl]-acetamid

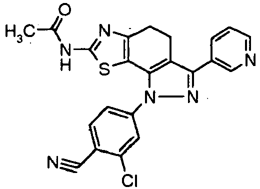

Eine Lösung von Z-1 (5.1 g, 16 mmol) und H-6 (4.3 g, 16 mmol) in 180 mL Eisessig wird 14 h bei 50 °C gerührt. Das Reaktionsgemisch wird eingeengt und anschließend auf Eiswasser gegeben. Der entstandene Niederschlag wird filtriert. Ausbeute: 5.2 g (w = 0.9).

### Z-4b) N-[1-(4-Cyano-3-dimethylamino-phenyl)-3-pyridin-3-yl-4,5-dihydro-1H-pyrazolo[4,3-g]benzthiazol-7-yl]-acetamid

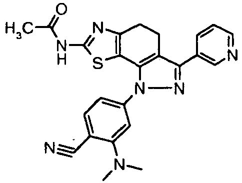

Eine Lösung von Z-4a (0.2 g, 0.33 mmol) und Dimethylamin (0.81 mL, 1.6 mmol) in 2 mL DMSO wird 15 min bei 130 °C in der Mikrowelle erhitzt und anschließend mit Wasser versetzt. Der entstandene Niederschlag wird abfiltriert und chromatographisch an Kieselgel mit Dichlormethan/Methanol gereinigt. Ausbeute: 82 mg.

Zu einer Lösung von Z-4b (100 mg, 0.22 mmol) in 1.5 mL Pyridin und 0.7 mL Eisessig wird Raney-Nickel (0.2 g, 3.4 mmol) zugegeben und anschließend 2 h bei 50 °C gerührt. Das Reaktionsgemisch wird über Celite filtriert und das Filtrat eingeengt. Der Rückstand wird chromatographisch mittels RP-HPLC gereinigt. Ausbeute: 53 mg.

### Z-5) 1-(2-Chlor-4-dimethylaminomethylphenyl)-3-pyridin-3-yl-4,5-dihydro-1H-pyrazolo[4,3-g]benzthiazol-7-ylamin

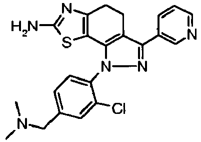

Zu einer Lösung von 4-(7-Amino-3-pyridin-3-yl-4,5-dihydro-pyrazolo[4,3-*g*]benzthiazol-1-yl)-3-chlor-*N,N*-dimethylbenzamid (PCT/EP05055021) (0.46 g, 1 mmol) in 25 mL trockenem THF wird unter Argonatmosphäre bei -78 °C eine Lösung von Lithiumaluminiumhydrid (1 M in THF, 3 mL) zugegeben. Nach 5 min wird das Kältebad entfernt und das Reaktionsgemisch auf RT erwärmt. Anschließend wird unter Kühlung 1 mL Wasser zugetropft und 30 min gerührt. Der Niederschlag wird filtriert und das Filtrat eingeengt. Ausbeute: 0.34 g.

### Z-6) 1-(4-Dimethylaminomethyl-phenyl)-3-pyridin-3-yl-4,5-dihydro-1H-pyrazolo[4,3-g]benzthiazol-7-ylamin

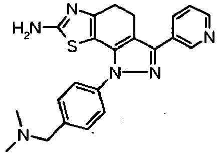

Analog der Darstellung von Z-5 wird ausgehend von 4-(7-Amino-3-pyridin-3-yl-4,5-dihydro-pyrazolo[4,3-*g*]benzthiazol-1-yl)-*N,N*-dimethyl-benzamid (PCT/EP05055021) (1.4 g, 3.4 mmol) und Lithiumaluminiumhydrid (1 M in THF, 6 mL) das gewünschte Produkt enthalten. Ausbeute: 1.4 g (w = 0.9).

### Z-7) 1-(2-Chlor-4-piperidin-1-ylmethylphenyl)-3-pyridin-3-yl-4,5-dihydro-1H-pyrazolo[4,3-g]benzthiazol-7-ylamin

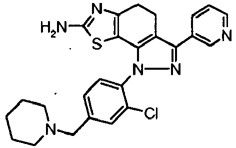

### Z-7a) [4-(7-Amino-3-pyridin-3-yl-4,5-dihydro-pyrazolo[4,3-g]benzthiazol-1-yl)-3-chlorphenyl]-piperidin-1-yl-methanon

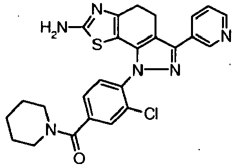

Zu einer Lösung von 4-(7-Amino-3-pyridin-3-yl-4,5-dihydro-pyrazolo[4,3-*g*]benzthiazol-1-yl)-3-chlorbenzoesäure (PCT/EP05055021) (0.25 g, 0.5 mmol) und *O*-(7-Azabenztriazol-1-yl)-*N,N,N,N'*-tetramethyluroniumhexafluorphosphat (0.24 g, 0.6 mmol) in 10 mL Dichlormethan wird *N,N*-Diisopropylethylamin (0.42 mL, 2.4 mmol) zugegeben und 1 h bei RT gerührt. Anschließend wird Piperidin (60 µL, 0.6 mmol) zugegeben und über Nacht bei RT gerührt. Das Reaktionsgemisch wird eingeengt, in 2 mL DMF gelöst und chromatographisch mittels RP-HPLC gereinigt. Ausbeute: 94 mg.

Zu einer Lösung von Z-7a (89 mg, 0.18 mmol) in 8 mL trockenem THF wird unter Argonatmosphäre Boran-THF-Komplex (1 M in THF, 1.81 mL) zugegeben und das Reaktionsgemisch 13 h bei 60 °C gerührt. Anschließend wird 5 mL TMEDA zugegeben und das Reaktionsgemisch eingeengt. Der Rückstand wird in 2 mL DMF gelöst, filtriert und chromatographisch mittels RP-HPLC gereinigt. Ausbeute: 15 mg.

### Z-8) N-[1-(2-Fluor-5-formylphenyl)-3-pyridin-3-yl-4,5-dihydro-1H-pyrazolo[4,3-g]benzthiazol-7-yl]-acetamid

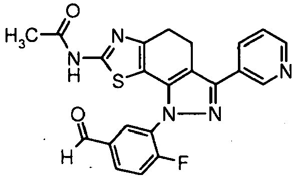

### Z-8a) N-[1-(5-Cyano-2-fluor-phenyl)-3-pyridin-3-yl-4,5-dihydro-1H-pyrazolo[4,3-g]benzthiazol-7-yl]-acetamid

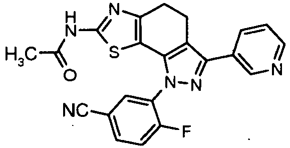

Analog zur Darstellung von I-1 wird ausgehend von Z-1 (4.7 g, 15 mmol), H-8 (4.1 g, 15.1 mmol) und 170 mL Eisessig die gewünschte Verbindung 1-9 erhalten. Ausbeute: 4.8 g (w = 0.8)

Eine Lösung von Z-8a (400 mg, w = 0.7, 0.65 mmol) in 3 mL Pyridin und 1.4 mL Essigsäure wird 30 min bei RT gerührt, mit Raney-Nickel (400 mg, 6.8 mmol) versetzt und 1 h bei 60 °C gerührt. Anschließend wird über Celite filtriert, mit Pyridin gewaschen und das Filtrat im Vakuum eingeengt. Der Rückstand wird in DMF aufgenommen und mittels RP-Chromatographie gereinigt. Ausbeute: 122 mg.

### Beispiel 1.1) N-[1-(4-Azetidin-1-ylmethyl-2-chlorphenyl)-3-pyridin-3-yl-4,5-dihydro-1H-pyrazolo[4,3-g]benzthiazol-7-yl]-acetamid

Eine Lösung von Z-3 (0.1 g, 0.16 mmol) und Azetidin (12 µL, 0.18 mmol) in 4 mL Dichlormethan wird 1.5 h bei RT gerührt. Anschließend wird Natriumtrisacetoxyborhydrid (40 mg, 19 mmol) zugegeben und 2 d bei RT gerührt. Das Reaktionsgemisch wird eingeengt, der Rückstand in 2 mL DMF gelöst und chromatographisch mittels RP-HPLC: gereinigt. Ausbeute: 28 mg.

Die Darstellung der Beispiele 1.2 - 1.8 erfolgt analog der Synthese von 2.1.

| # | Edukt | Struktur | Masse [M+l]⁺ | HPLC:Rt [min] |
|---|---|---|---|---|
| 1.1 | Z-3 | 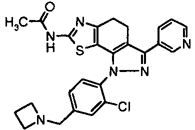 | 491 | 0.58 |
| 1.2 | Z-3 | 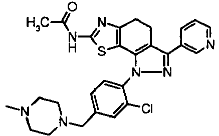 | 534 | 1.30 |
| 1.3 | Z-3 | 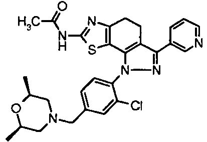 | 549 | 1.33 |
| 1.4 | Z-3 | 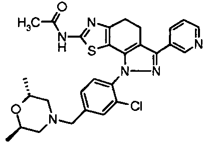 | 549 | 1.35 |
| 1.5 | Z-3 | 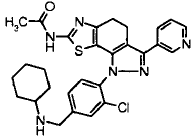 | 533 | 0.74 |
| 1.6 | Z-3 | 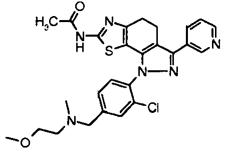 | 523 | 1.27 |
| 1.7 | Z-3 | 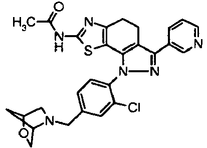 | 533 | 0.51 |
| 1.8 | Z-4 | 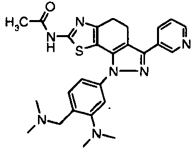 | 488 | 1.35 |

### Beispiel 2.1) N-{1-[4-(1-Morpholin-4-yl-cyclopropyl)-phenyl]-3-pyridin-3-yl-4,5-dihydro-1H-pyrazolo[4,3-g]benzthiazol-7-yl}-acetamid

Eine Lösung von Z-1 (0.13 g, 0.4 mmol) und H-4 (0.18 g, 0.52 mmol) in 5 mL Eisessig wird über Nacht bei RT gerührt. Das Reaktionsgemisch wird eingeengt, der Rückstand in 2 mL DMSO gelöst und mittels RP-HPLC gereinigt. Ausbeute: 15 mg.

Die Darstellung der Beispiele 2.2 - 2.5 erfolgt analog der Synthese von 2.1.

| # | Edukt | Struktur | Masse [M+1]⁺ | HPLC:Rt [min] |
|---|---|---|---|---|
| 2.1 | Z-1 | 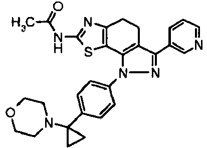 | 513 | 1.42 |
| | H-4 | | | |
| 2.2 | Z-1 | 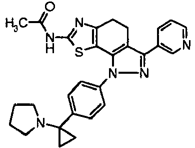 | 497 | 1.42 |
| | H-3 | | | |
| 2.3 | Z-1 | 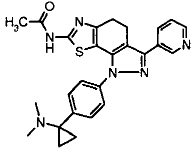 | 471 | 1.23 |
| | H-2 | | | |
| 2.4 | Z-2 | 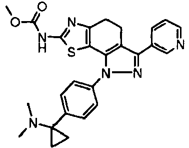 | 487 | 1.27 |
| | H-2 | | | |
| 2.5 | Z-1 | 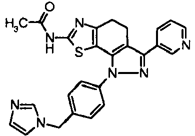 | 468 | 1.22 |
| | H-5 | | | |

### Beispiel 2.6) N-[1-(2-Chlor-4-morpholin-4-ylmethyl-phenyl)-3-pyridin-3-yl-4,5-dihydro-1H-pyrazolo[4,3-g]benzthiazol-7-yl]-acetamid

Eine Lösung von Z-1 (4.6 g, 15 mmol) und H-1 (4.7 g, 15 mmol) in 54 mL Ethanol wird in drei Teilen 10 - 12 min bei 120 -135 °C in der Mikrowelle erhitzt. Der entstandene Niederschlag wird filtriert und mit Ethanol gewaschen. Ausbeute: 4.3 g.

Die Darstellung von Beispiel 2.7 erfolgt analog der Synthese von 2.6.

| # | Edukt | Struktur | Masse [M+1]⁺ | HPLC:Rt [min] |
|---|---|---|---|---|
| 2.6 | Z-1 | 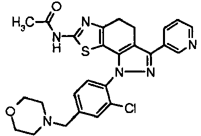 | 521 | 0.55 |
| | H-1 | | | |
| 2.7 | Z-2 | 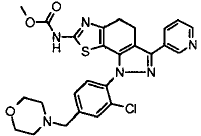 | 537 | 1.26 |
| | H-1 | | | |

### Beispiel 3.1) N-[1-(2-Chlor-4-dimethylaminomethyl-phenyl)-3-pyridin-3-yl-4,5-dihydro-1H-pyrazolo[4,3-g]benzthiazol-7-yl]-acetamid

Zu einer Lösung von Z-5 (0.18 g, 0.4 mmol) in 50 mL Dichlormethan werden Pyridin (0.4 mL, 40 mmol) und Acetylchlorid (0.32 mL, 4 mmol) zugegeben und das Reaktionsgemisch 1 h bei RT gerührt. Anschließend wird 2 mL Methanol zugegeben und das Reaktionsgemisch eingeengt. Der Rückstand wird in 2 mL DMSO gelöst und chromatographisch mittels RP-HPLC und anschließend an Kieselgel mit Dichlormethan/Methanol gereinigt. Ausbeute: 35 mg.

Die Darstellung von Beispiel 3.2 und 3.3 erfolgt analog der Synthese von 3.1.

| # | Edukt | Struktur | Masse [M+1]⁺ | HPLC:Rt [min] |
|---|---|---|---|---|
| 3.1 | Z-5 | 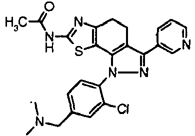 | 479 | 0.50 |
| 3.2 | Z-6 | 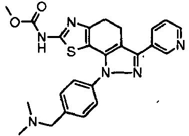 | 461 | 1.28 |
| 3.3 | Z-7 | 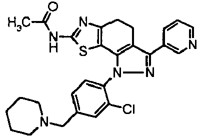 | 519 | 1.32 |

### Beispiel 4) 1-[1-(2-Chlor-4-dimethylaminomethyl-phenyl)-3-pyridin-3-yl-4,5-dihydro-1H-pyrazolo[4,3-g]benzthiazol-7-yl]-3-methyl-harnstoff

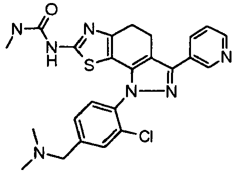

### 4-a) 3-Chlor-4-(7-ethylsulfanylcarbonylamino-3-pyridin-3-yl-4,5-dihydro-pyrazolo[4,3-g]benzthiazol-1-yl)-benzoesäuremethylester

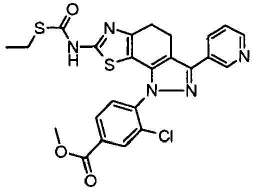

Eine Lösung von [7-Oxo-6-(pyridin-3-carbonyl)-4,5,6,7-tetrahydro-benzthiazol-2-yl]-thiocarbamidsäure-*S*-ethylester (PCT/EP05055021) (3.6 g, 10 mmol) und 3-Chlor-4-hydrazino-benzoesäuremethylester (3.7 g, 18 mmol) in 100 mL Eisessig wird über Nacht bei 50 °C gerührt, anschließend in Wasser gegeben und der entstandene Niederschlag abfiltriert. Ausbeute 2.9 g.

### 4-b) 3-Chlor-4-(7-ethylsulfanylcarbonylamino-3-pyridin-3-yl-4,5-dihydro-pyrazolo[4,3-g]benzthiazol-1-yl)-benzoesäure

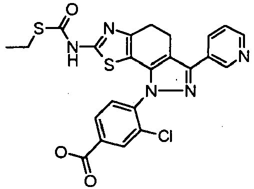

Ein Suspension von 4-a (5.3 g, 10 mmol) in 20 mL Dioxan wird mit Lithiumhydroxid (3.2 g, in 50 mL Wasser) versetzt. Nach Aufklaren der Lösung wird die Reaktionsmischung weitere 30 min bei RT gerührt und mit 5 M Salzsäure versetzt. Der entstandene Niederschlag wird filtriert, mit Wasser gewaschen und getrocknet. Ausbeute: 3.8 g.

### 4-c) [1-(2-Chlor-4-dimethylcarbamoylphenyl)-3-pyridin-3-yl-4,5-dihydro-1H-pyrazolo[4,3-g]benzthiazol-7-yl]-thiocarbamidsäure-S-ethyl ester

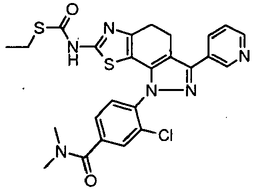

Zu einer Lösung von 4-b (0.52 g, 1 mmol), *O*-Benztriazol-1-yl-*N,N,N;N'-*tetramethyluroniumtetrafluorborat (0.38 g, 1.1 mmol) und *N,N*-Diisopropylethylamin (0.6 mL, 6.3 mmol) in 10 mL THF wird Dimethylamin (2 M in THF, 0.6 mL) gegeben und über Nacht bei RT gerührt. Anschließend wird das Reaktionsgemisch mit Wasser versetzt und mit Ethylacetat extrahiert. Die vereinigten organischen Phasen werden getrocknet und eingeengt. Ausbeute: 0.54 g.

### 4-d)3-Chlor-N,N-dimethyl-4-[7-(3-methyl-ureido)-3-pyridin-3-yl-4,5-dihydropyrazolo[4,3-g]benzthiazol-1-yl]-benzamid

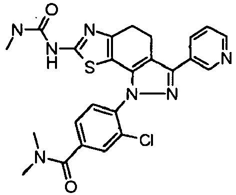

Eine Lösung von 4-c (0.5 g, 0.46 mmol) und Methylamin (2 M in THF, 2 mL) in 40 mL Dichlormethan/THF wird über Nacht bei 80 °C gerührt. Das Reaktionsgemisch wird eingeengt, der Rückstand in DMF gelöst und chromatographisch mittels RP-HPLC gereinigt. Ausbeute: 0.10 g.

Zu einer Lösung von 4-d (0.1 g, 0.2 mmol) in 10 mL trocknem THF wird unter Argonatmosphäre bei -78 °C Lithiumaluminumhydrid (1 M in THF, 0.5 mL) zugegeben. Nach Entfernen des Kältebads wird das Reaktionsgemisch 1.5 h bei RT gerührt und anschließend mit Wasser versetzt. Das Reaktionsgemisch eingeengt, der Rückstand in DMF gelöst und chromatographisch mittels RP-HPLC gereinigt. Ausbeute: 30 mg. HPLC: Rt = 0.59 min

[M+1]⁺ = 494

### Beispiel 5.1 N-{1-[2-Fluor-5-(4-methylpiperazin-1-ylmethyl)-phenyl]-3-pyridin-3-yl-4,5-dihydro-1H-pyrazolo[4,3-g]benzthiazol-7-yl}-acetamid

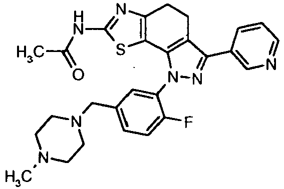

Eine Suspension von Z-8 (50 mg, 0.11 mmol) in 1 mL 1,2-Dichlorethan wird mit *N-*Methylpiperazin (19 mL, 0.17 mmol) versetzt und 1 h bei RT gerührt. Anschließend werden nacheinander 10 µL Eisessig und Natriumtrisacetoxyborhydrid (37 mg, 0.17 mmol) zugegeben. Nach vollständigem Umsatz (HPLC) wird die Reaktionsmischung chromatographisch gereinigt. Ausbeute: 32 mg.

### Beispiel 5.2

Analog zu Beispiel 5.1 wird folgende Beispiele synthetisiert

| # | Edukt | Struktur | Masse [M+1]⁺ | HPLC Rt [min] |
|---|---|---|---|---|
| 5.1 | Z-8 | 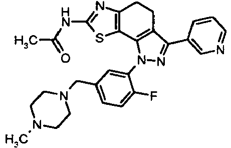 | 518 | 1.33 |
| 5.2 | Z-8 | 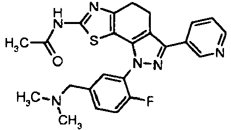 | 463 | 1.25 |

Weiters können nach den vorstehend beschriebenen Methoden folgende Verbindungen hergestellt werden.

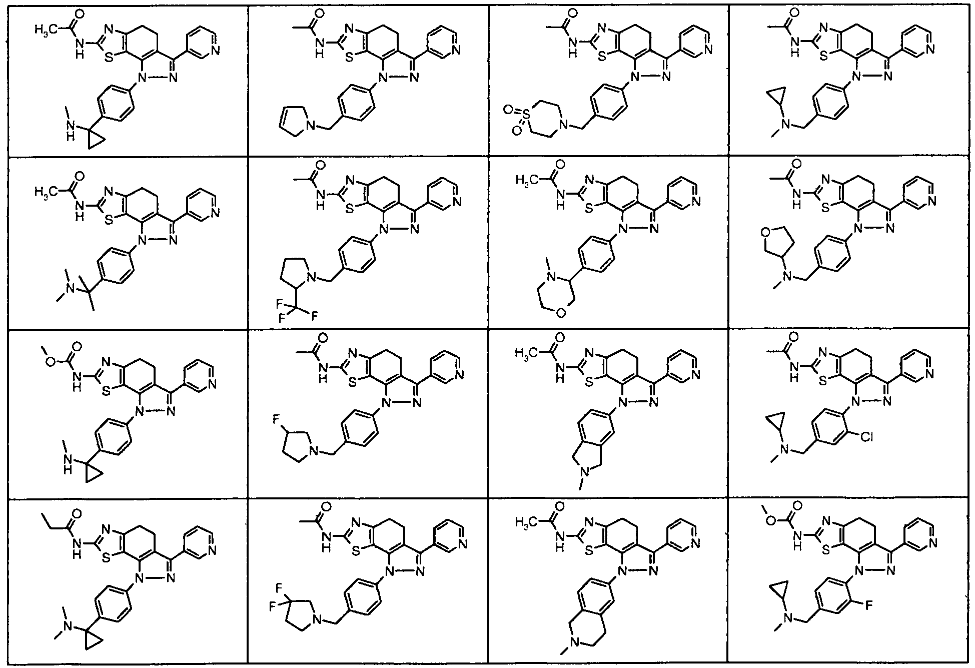

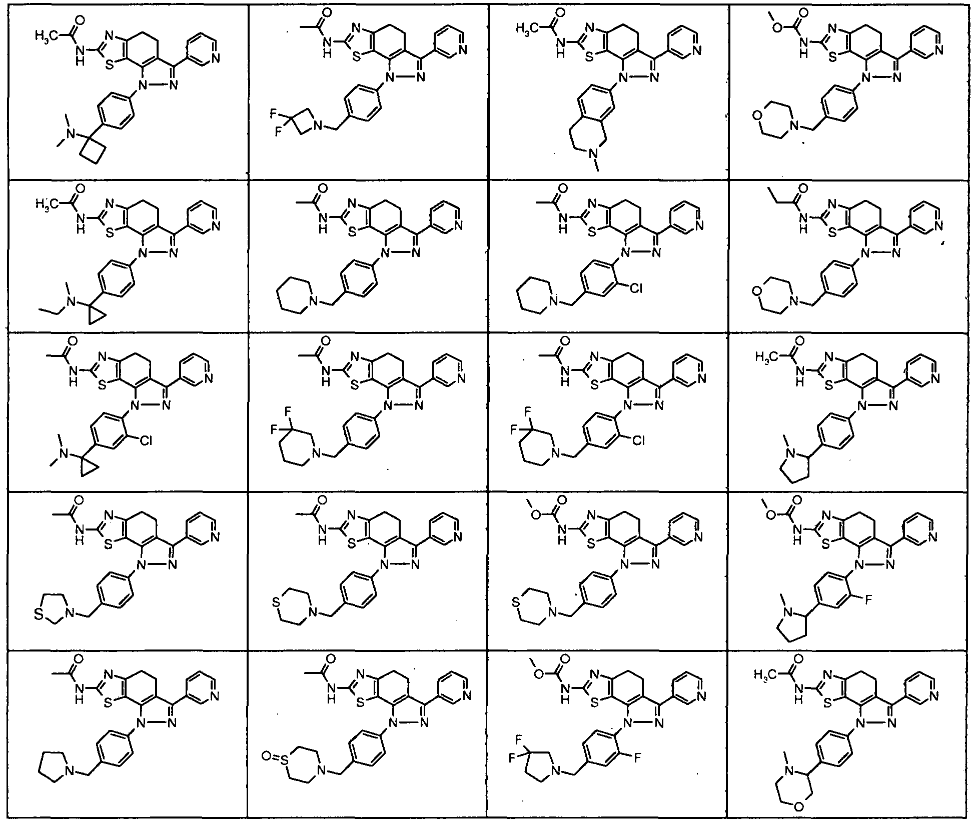

Das nachfolgende Beispiel beschreibt die biologische Wirkung der erfindungsgemäßen Verbindungen ohne die Erfindung auf dieses Beispiel einzuschränken.

### HCT116 Zytotoxizitätstest

Der Test basiert auf der Reduktion von AlamarBlue (Biosource Int., USA) in lebenden (metabolisch aktiven) Zellen zu einem fluorometrisch nachweisbaren Produkt. In Gegenwart von zelltoxischen Substanzen kann das Substrat nicht mehr reduziert werden, so dass kein Anstieg der Fluoreszenz messbar ist.

HCT116 (humane Kolonkarzinomzelllinie) Zellen werden in Mikrotiterplatten ausgesät und über Nacht in Kulturmedium bei 37 °C und 5% CO₂ inkubiert. Die Testsubstanzen werden in Medium schrittweise verdünnt und zu den Zellen gegeben, so dass das Gesamtvolumen 200 µL/well beträgt. Als Kontrollen dienen Zellen, die mit Medium, jedoch nicht mit Substanz, versetzt werden. Nach einer Inkubationszeit von 4 - 6 Tagen gibt man 20 µL/well AlamarBlue zu und inkubiert die Zellen für weitere 6 - 8 h bei 37 °C. Zur Fluoreszenzmessung wird bei einer Wellenlänge von 545 nm angeregt und bei 590 nm die Emission gemessen.

Die Berechnung von EC₅₀ Werten erfolgt mit Hilfe des Programms GraphPad Prism. Alle Verbindungen der angeführten Beispiele 1.1 bis 5.2 weisen einen EC₅₀ (HCT-116) von kleiner als 5 µM auf.

Die Substanzen der vorliegenden Erfindung sind PI3-Kinase Inhibitoren. Aufgrund ihrer biologischen Eigenschaften eignen sich die neuen Verbindungen der allgemeinen Formel (1), deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphome und solide Tumore; HautErkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001).

Beispielsweise können folgende Krebserkrankungen mit erfindungsgemäßen Verbindungen behandelt werden, ohne jedoch darauf beschränkt zu sein: Hirntumore wie beispielsweise Akustikusneurinom, Astrozytome wie pilozytisches Astrozytome, fibrilläres Astrozytom, protoplasmatisches Astrozytom, gemistozytäres Astrozytom, anaplastisches Astrozytom und Glioblastome, Hirnlymphome, Hirnmetastasen, Hypophysentumor wie Prolaktinom, HGH (human growth hormon) produzierender Tumor und ACTH produzierender Tumor (adrenocorticotropes Hormon), Kraniopharyngiome, Medulloblastome, Meningeome und Oligodendrogliome; Nerventumore (Neoplasmen) wie zum Beispiel Tumore des vegetativen Nervensystems wie Neuroblastoma sympathicum, Ganglioneurom, Paragangliom (Phäochromozytom, Chromaffinom) und Glomuscaroticum-Tumor, Tumore am peripheren Nervensystem wie Amputationsneurom, Neurofibrom, Neurinom (Neurilemmom, Schwannom) und malignes Schwannom, sowie Tumore am zentralen Nervensystem als Hirn- und Rückenmarkstumoren; Darmkrebs wie zum Beispiel Rektumkarzinom, Kolonkarzinom, Analkarzinom, Dünndarmtumore und Zwölffingerdarmtumore; Lidtumore wie Basaliom oder Basalzellkarzinom; Bauchspeicheldrüsenkrebs oder Pankreaskarzinom; Blasenkrebs oder Blasenkarzinom; Lungenkrebs (Bronchialkarzinom) wie beispielsweise kleinzellige Bronchialkarzinome (Haferzellkarzinome) und nicht-kleinzellige Bronchialkarzinome wie Plattenepithelkarzinome, Adenokarzinome und großzellige Bronchialkarzinome; Brustkrebs wie zum Beispiel Mammakarzinom wie infiltrierend duktales Karzinom, Kolloidkarzinom, lobulär invasives Karzinom, tubuläres Karzinom, adenozystisches Karzinom und papilläres Karzinom; Non-Hodgkin-Lymphomen (NHL) wie beispielsweise Burkitt-Lymphom, niedrigmaligne Non-Hodgkin-Lymphomen (NHL) und Mucosis fungoides; Gebärmutterkrebs oder Endometriumkarzinom bzw. Corpuskarzinom; CUP-Syndrom (Cancer of Unknown Primary); Eierstockskrebs oder Ovarial-Karzinom wie mucinöse, endometriale oder seröse Krebs; Gallenblasenkrebs; Gallengangskrebs wie beispielsweise Klatskin-Tumor; Hodenkrebs wie zum Beispiel Seminome und Nicht-Seminome; Lymphom (Lymphosarkom) wie zum Beispiel malignes Lymphom, Morbus Hodgkin, Non-Hodgkin-Lymphomen (NHL) wie chronisch lymphatische Leukämie, Haarzell-Leukämie, Immunozytom, Plasmozytom (multiples Myelom), Immunoblastom, Burkitt-Lymphom, T-Zonen-Mycosis fungoides, großzellig-anaplastisches Lymphoblastom und Lymphoblastom; Kehlkopfkrebs wie zum Beispiel Stimmbandtumoren, supraglottisch, glottisch und subglottisch Kehlkopftumore; Knochenkrebs wie zum Beispiel Osteochondrom, Chondrom, Chondroblastom, Chondromyxoidfibrom, Osteom, Osteoid-Osteom, Osteoblastom, eosinophiles Granulom, Riesenzell-Tumor, Chondrosarkom, Osteosarkom, Ewing-Sarkom, Retikulo-Sarkom, Plasmozytom, Riesenzell-Tumor, fibröse Dysplasie, juvenile Knochenzyste und aneurysmatische Knochenzyste; Kopf-Hals-Tumoren wie zum Beispiel Tumore der Lippen, Zunge, Mundboden, Mundhöhle, Zahnfleisch, Gaumen, Speicheldrüsen, Rachen, Nasenhöhlen, Nasennebenhöhlen, Kehlkopf und Mittelohr; Leberkrebs wie zum Beispiel das Leberzellkarzinom oder hepatozelluläres Karzinom (HCC); Leukämien wie zum Beispiel akute Leukämien wie akute lymphatische/lymphoblastische Leukämie (ALL), akute myeloische Leukämie (AML); chronische Leukämien wie chronisch lymphatische Leukämie (CLL), chronisch myeloische Leukämie (CML); Magenkrebs oder Magenkarzinom wie zum Beispiel papilläres, tubuläres und muzinöses Adenokarzinom, Siegelringzellkarzinom, adenosquamöses Karzinom, kleinzelliges Karzinom und undifferenziertes Karzinom; Melanome wie zum Beispiel oberflächlich spreitendes, knotiges, lenitgo-maligna und akral-lentiginöse Melanom; Nierenkrebs wie zum Beispiel Nierenzellkarzinom oder Hypernephrom oder Grawitz-Tumor; Speiseröhrenkrebs oder Ösophaguskarzinom; Peniskrebs; Prostatakrebs; Rachenkrebs oder Pharynxkarzinome wie zum Beispiel Nasopharynxkarzinome, Oropharynxkarzinome und Hypopharynadcarzinome; Retinoblastom; Scheidenkrebs oder Vaginalkarzinom; Plattenepithelkarzinome, Adenokarzinome, in situ Karzinome, maligne Melanome und Sarkome; Schilddrüsen-Karzinome wie zum Beispiel papilläre, follikuläre und medulläre Schilddrüsen-Karzinom, sowie anaplastische Karzinome; Spinaliom, Stachelzellkarzinom und Plattenepithelkarzinom der Haut; Thymome, Urethrakrebs und Vulvakrebs.

Die neuen Verbindungen können zur Prävention, Kurz- oder Langzeitbehandlung der vorstehend erwähnten Krankheiten auch gegebenenfalls in Kombination mit anderen "State-of-art" Verbindungen, wie anderen anti-Tumor Substanzen, zytotoxischen Substanzen, Zellproliferationshemmern, anti-angiogenen Substanzen, Steroiden oder Antikörpern, verwendet werden.

Die Verbindungen der allgemeinen Formel (1) können allein oder in Kombination mit anderen erfindungsgemäßen Wirkstoffen, gegebenenfalls auch in Kombination mit weiteren pharmakologisch aktiven Wirkstoffen, zur Anwendung gelangen. Chemotherapeutika, welche in Kombination mit den erfindungsgemäßen Verbindungen verabreicht werden können, umfassen, ohne darauf eingeschränkt zu sein, Hormone, Hormonanaloge und Antihormone (z.B. Tamoxifen, Toremifen, Raloxifen, Fulvestrant, Megestrol Acetat, Flutamid, Nilutamid, Bicalutamid, Aminoglutethimid, Cyproteron Acetat, Finasterid, Buserelin Acetat, Fludrocortinson, Fluoxymesteron, Medroxyprogesteron, Octreotid), Aromatase Inhibitoren (z.B. Anastrozol, Letrozol, Liarozol, Vorozol, Exemestan, Atamestan,), LHRH Agonisten und Antagonists (z.B. Goserelin Acetat, Luprolid), Inhibitoren von Wachstumsfaktoren (growth factors wie zum Beispiel "platelet derived growth factor" und "hepatocyte growth factor", Inhibitoren sind z. B. "growth factor"-Antikörper, "growth factor Receptor"-Antikörper und Tyrosinekinase Inhibitoren, wie zum Beispiel Gefitinib, Imatinib, Lapatinib, Erbitux® und Trastuzumab); Antimetabolite (z.B. Antifolate wie Methotrexat, Raltitrexed, Pyrimidinanaloge wie 5-Fluorouracil, Capecitabin und Gemcitabin, Purin- und Adenosinanaloge wie Mercaptopurin, Thioguanin, Cladribin und Pentostatin, Cytarabin, Fludarabin); Antitumor-Antibiotica (z.B. Anthracycline wie Doxorubicin, Daunorubicin, Epirubicin und Idarubicin, Mitomycin-C, Bleomycin, Dactinomycin, Plicamycin, Streptozocin); Platinderivative (z.B. Cisplatin, Oxaliplatin, Carboplatin); Alkylierungsagentien (z.B. Estramustin, Meclorethamin, Melphalan, Chlorambucil, Busulphan, Dacarbazin, Cyclophosphamid, Ifosfamid, Temozolomid, Nitrosoharnstoffe wie zum Beispiel Carmustin und Lomustin, Thiotepa); antimitotische Agentien (z.B. Vinca-Alkaloide wie zum Beispiel Vinblastin, Vindesin, Vinorelbin und Vincristin; und Taxane wie Paclitaxel, Docetaxel); Topoisomerase Inhibitoren (z.B. Epipodophyllotoxine wie zum Beispiel Etoposid und Etopophos, Teniposid, Amsacrin, Topotecan, Irinotecan, Mitoxantron) und verschiedene Chemotherapeutica wie Amifostin, Anagrelid, Clodronat, Filgrastin, Interferon alpha, Leucovorin, Rituximab, Procarbazin, Levamisol, Mesna, Mitotan, Pamidronat und Porfimer.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, das heißt in Mengen die ausreichend sind, um den unten angegebenen Dosierungsbereich zu erreichen. Die genannten Dosen können, falls erforderlich, mehrmals täglich gegeben werden.

entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserem oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken:

Pharmazeutische Formulierungsbeispiele

| A) | Tabletten | pro Tablette |
|---|---|---|
| | Wirkstoff nach Formel (1) | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff nach Formel (1) | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff nach Formel (1) | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (1), 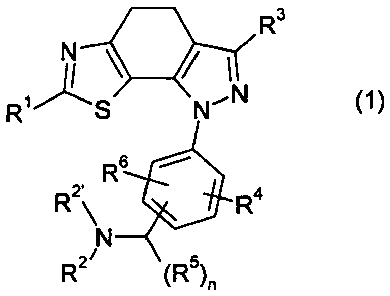 worin
**R¹** ausgewählt aus der Gruppe bestehend aus -NHR^{a}, -NHC(O)R^{a}, -NHC(O)OR^{a}, -NHC(O)NR^{a}R^{a} und -NHC(O)SR^{a}, und
**R²** und **R^{2'}** jeweils unabhängig voneinander Wasserstoff oder ein Rest, ausgewählt aus der Gruppe bestehend aus C₁₋₆Alkyl, C₃₋₈Cycloalkyl, C₆₋₁₀Aryl, 5-10 gliedriges Heteroaryl und 3-8 gliedriges Heterocycloalkyl, gegebenenfalls substituiert mit einem oder mehreren R^{a} und/oder R^{b}, oder
**R²** und **R^{2'}** gemeinsam mit dem eingeschlossenen Stickstoffatom einen Heterocycloalkyl- oder Heteroarylring bilden, welcher gegebenenfalls ein oder mehrere weitere Heteroatome enthalten kann, ausgewählt aus der Gruppe bestehend aus N, O und S und gegebenenfalls mit einem oder mehreren R^{b} und/oder R^{d} substituiert sein kann, und
**R³** ein Rest ausgewählt aus der Gruppe bestehend aus 5-6 gliedriges Heteroaryl, gegebenenfalls substituiert mit einem oder mehreren, gleich oder verschiedenen R^{c} und/oder R^{b}, und
**R⁴** und **R⁶** jeweils unabhängig voneinander Wasserstoff oder ein Rest ausgewählt aus der Gruppe bestehend aus Halogen, -CF₃, -OCF₃, -CN, -NR^{c}R^{c}, -SR^{c}, -S(O)R^{c}, -S(O)₂R^{c} und -OR^{c}, oder C₁₋₃Alkyl gegebenenfalls substituiert mit Fluor, -CN, -NR^{f}R^{f} und/oder -OR^{f}, und
**R⁵** C₁₋₃Alkyl, oder
zwei **R⁵** gemeinsam mit dem eingeschlossenen Kohlenstoffatom einen C₃₋₈Cycloalkylring oder ein 3-8 gliedriges Heterocycloalkyl bilden, und
**n** gleich 0, 1 oder 2, oder
**R²** mit einem **R⁵** und dem eingeschlossenen Stickstoff- und Kohlenstoffatom gemeinsam einen 4-8 gliedrigen Heterocycloalkylring bilden, oder
**R²** mit einem geeigneten **R⁶** und den eingeschlossenen Stickstoff- und Kohlenstoffatomen gemeinsam einen **4-8** gliedrigen Heterocycloalkylring bilden, und jedes **R^{a}** unabhängig voneinander Wasserstoff oder ein gegebenenfalls mit einem oder mehreren, gleich oder verschiedenen R^{b} und/oder R^{c} substituierter Rest ausgewählt aus der Gruppe bestehend aus C₁₋₆Alkyl, C₃-₈Cycloalkyl, C₄-₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und
jedes **R^{b}** ein geeigneter Rest jeweils unabhängig voneinander ausgewählt aus der Gruppe bestehend aus =O, -OR^{c}, C₁₋₃Haloalkyloxy, =S, -SR^{c}, =NR^{c}, =NOR^{c}, -NR^{c}R^{c}, Halogen, -CF₃, -CN, -NC, -NO₂, -N₃, -S(O)R^{c}, -S(O)₂R^{c}, -S(O)₂OR^{c}, -S(O)NR^{c}R^{c}, - S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, - C(O)OR^{c}, -C(O)NR^{c}R^{c}, -C(O)N(R^{d})OR^{c} -CN(R^{d})NR^{c}R^{c}, -OC(O)R^{c}, -OC(O)OR^{c}, - OC(O)NR'R', -OCN(R^{d})NR^{c}R^{c}, -N(R^{d})C(O)R^{c}, -N(R^{d})C(S)R^{c}, -N(R^{d})S(O)2R^{c}, - N(R^{d})C(O)OR^{c}, -N(R^{d})C(O)NR^{c}R^{c}, und -N(R^{d})CN(R^{d})NR^{c}R^{c}, und
jedes **R^{c}** unabhängig voneinander Wasserstoff oder ein gegebenenfalls mit einem oder mehreren, gleich oder verschiedenen R^{d} substituierter Rest ausgewählt aus der Gruppe bestehend aus C₁₋₆Alkyl, C₃₋₈Cycloalkyl, C₄-₁₁Cycloalkylalkyl, C₆₋₁₀Aryl, C₇-1₆Arylalkyl, 2-6 gliedriges Heteroalkyl, **3-8** gliedriges Heterocycloalkyl, 4-14 gliedriges Heterocycloalkylalkyl, 5-10 gliedriges Heteroaryl und 6-16 gliedriges Heteroarylalkyl, und
jedes **R^{d}** unabhängig voneinander Wasserstoff, C₁₋₆Alkyl, C₃₋₈Cycloalkyl, C₄₋₁₁Cycloalkylalkyl, C₆₋₁₀Aryl, C₇₋₁₆Alylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl bedeuten,
wobei unter Alkyl-Substituenten jeweils gesättigte, ungesättigte, geradkettige oder verzweigte aliphatische Kohlenwasserstoffreste (Alkylrest) zu verstehen sind und sowohl gesättigte Alkylreste als auch ungesättigte Alkenyl- und Alkinylreste umfasst; und
unter Cycloalkyl ein mono- oder bizyklischer Ring zu verstehen ist, wobei das Ringsystem ein gesättigter Ring aber auch ein ungesättigter, nichtaromatischer Ring sein kann, welcher gegebenfalls auch Doppelbindungen enthalten kann;
gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze mit der Maßgabe, dass folgende Verbindungen nicht umfasst sind:
*N*-[1-(4-Morpholin-4-ylmethyl-phenyl)-3-pyridin-2-yl-4,5-dihydro-1*H*-pyrazolo[4,3-*g*]benzthiazol-7-yl]-acetamid,
*N*-[1-(4-Dimethylaminomethyl-phenyl)-3-pyridin-3-yl-4,5-dihydro-1*H* pyrazolo[4,3-*g*]benzthiazol-7-yl]-acetamid,
*N*-{1-[4-(Benzylamino-methyl)-2-chloro-phenyl]-3-pyrazin-2-yl-4,5-dihydro-1*H-*pyrazolo[4,3-*g*]benzthiazol-7-yl}-acetamid und
*N*-(1-(2-Chloro-4-[(1-cyclopentyl-piperidin-4-ylamino)-methyl]-phenyl)-3-pyrazin-2-yl-4,5-dihydro-1*H*-pyrazolo[4,3-*g*]benahiazol-7-yl)-acetarnid.

2. Verbindungen der allgemeinen Formel (1A), 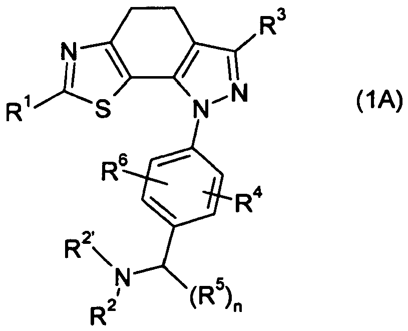 worin die Substituenten wie in Anspruch 1 definiert sind.

3. Verbindungen gemäß einem der Ansprüche 1 oder 2, worin **R³** unsubstituiertes Pyridyl bedeutet.

4. Verbindungen gemäß einem der Ansprüche 1 bis 3, worin **R³** unsubstituiertes Pyridin-3-yl bedeutet.

5. Verbindungen gemäß einem der Ansprüche 1 bis 4, worin **R¹** ausgewählt ist aus der Gruppe bestehend aus -NHC(O)R^{a}, -NHC(O)OR^{a} und -NHC(O)NR^{a}R^{a}.

6. Verbindungen, oder deren pharmakologisch verträglichen Salze, gemäß einem der Ansprüche 1 bis 5 als Arzneimittel.

7. Verbindungen, oder deren pharmakologisch verträglichen Salze, gemäß einem der Ansprüche 1 bis 5 zur Herstellung eines Arzneimittels mit antiproliferativer Wirkung.

8. Pharmazeutische Zubereitungen, enthaltend als Wirkstoff eine oder mehrere Verbindungen der allgemeinen Formel (1) gemäß einem der Ansprüche 1 bis 5 oder deren pharmakologisch verträglichen Salze, gegebenenfalls in Kombination mit üblichen Hilfs- und/oder Trägerstoffen.

9. Verwendung von Verbindungen der allgemeinen Formel (1) gemäß einem der Ansprüche 1 bis 5 zur Herstellung eines Arzneimittels zur Behandlung und/oder Prävention von Krebs.

10. Pharmazeutische Präparation umfassend eine Verbindung der allgemeinen Formel (1) gemäß einem der Ansprüche 1 bis 5 und mindestens eine weitere zytostatische oder zytotoxische, von Formel (1) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze.

## Claims

1. Compounds of general formula (1), 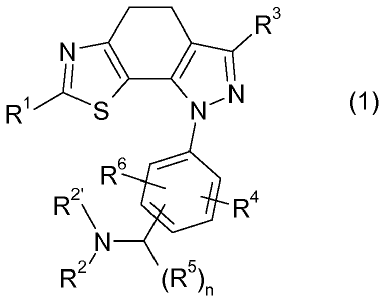 wherein
**R¹** is selected from among -NHR^{a}, -NHC(O)R^{a}, -NHC(O)OR^{a}, -NHC(O)NR^{a}R^{a} and -NHC(O)SR^{a}, and
**R²** and **R^{2'}** each independently of one another denote hydrogen or a group selected from among C₁₋₆alkyl, C₃₋₈cycloalkyl, C₆₋₁₀aryl, 5-10 membered heteroaryl and 3-8 membered heterocycloalkyl, optionally substituted by one or more **R^{a}** and/or **R^{b}**, or
**R²** and **R^{2'}** together with the enclosed nitrogen atom form a heterocycloalkyl or heteroaryl ring, which may optionally contain one or more further hetero atoms selected from among N, O and S and may optionally be substituted by one or more R^{b} and/or R^{d}, and
**R³** denotes a group selected from among 5-6 membered heteroaryl, optionally substituted by one or more identical or different R^{c} and/or R^{b}, and
**R⁴** and **R⁶** each independently of one another denote hydrogen or a group selected from among halogen, -CF₃, -OCF₃, -CN, -NR^{c}R^{c}, -SR^{c}, -S(O)R^{c}, -S(O)₂R^{c} and -OR^{c}, or C₁₋₃alkyl optionally substituted by fluorine, -CN, -NR^{f}R^{f} and/or -OR^{f}, and
**R⁵** denotes C₁₋₃alkyl, or
two **R⁵** together with the enclosed carbon atom form a C₃₋₈cycloalkyl ring or a 3-8 membered heterocycloalkyl, and
**n** is equal to 0, 1 or 2, or
**R²** together with an **R⁵** and the enclosed nitrogen and carbon atom form a 4-8 membered heterocycloalkyl ring, or
**R²** together with a suitable **R⁶** and the enclosed nitrogen and carbon atoms form a 4-8 membered heterocycloalkyl ring, and
each **R^{a}** independently of one another denotes hydrogen or a group optionally substituted by one or more identical or different R^{b} and/or R^{c} selected from among C₁₋₆alkyl, C₃₋₈cydoalkyl, C₄₋₁₁cycloalkylalkyl, C₆₋₁₀aryl, C₇₋₁₆arylalkyl, 2-6 membered heteroalkyl, 3-8 membered heterocycloalkyl, 4-14 membered heterocycloalkylalkyl, 5-10 membered heteroaryl and 6-16 membered heteroarylalkyl, and
each **R^{b}** denotes a suitable group each selected independently of one another from among =O, -OR^{c}, C₁₋₃haloalkyloxy, =S, -SR^{c}, =NR^{c}, =NOR^{c}, -NR^{c}R^{c}, halogen, -CF₃, -CN, -NC, -NO₂, -N₃, -S(O)R^{c}, -S(O)₂R^{c}, -S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{d})OR^{c} -CN(R^{d})NR^{c}R^{c}, -OC(O)R^{c}, -OC(O)OR^{c}, -OC(O)NR^{c}R^{c}, -OCN(R^{d})NR^{c}R^{c}, -N(R^{d})C(O)R^{c}, -N(R^{d})C(S)R^{c}, -N(R^{d})S(O)₂R^{c}, -N(R^{d})C(O)OR^{c}, -N(R^{d})C(O)NR^{c}R^{c}, and -N(R^{d})CN(R^{d})NR^{c}R^{c}, and
each **R^{c}** independently of one another denote hydrogen or a group optionally substituted by one or more identical or different R^{d} selected from among C₁₋₆alkyl, C₃₋₈cycloalkyl, C₄₋₁₁cycloalkylalkyl, C₆₋₁₀aryl, C₇₋₁₆arylalkyl, 2-6 membered heteroalkyl, 3-8 membered heterocycloalkyl, 4-14 membered heterocycloalkylalkyl, 5-10 membered heteroaryl and 6-16 membered heteroarylalkyl, and
each **R^{d}** independently of one another denotes hydrogen, C₁₋₆alkyl, C₃₋₈cycloalkyl, C₄₋₁₁cycloalkylalkyl, C₆₋₁₀aryl, C₇₋₁₆arylalkyl, 2-6 membered heteroalkyl, 3-8 membered heterocycloalkyl, 4-14 membered heterocycloalkylalkyl, 5-10 membered heteroaryl and 6-16 membered heteroarylalkyl,
wherein by alkyl substituents are meant saturated, unsaturated, straight-chain or branched aliphatic hydrocarbon groups (alkyl group) and the term encompasses both saturated alkyl groups and unsaturated alkenyl and alkynyl groups; and
by cycloalkyl is meant a mono- or bicyclic ring, while the ring system may be a saturated ring but also an unsaturated non-aromatic ring which may optionally also contain double bonds;
optionally in the form of the tautomers, the racemates, the enantiomers, the diastereomers and the mixtures thereof, as well as optionally the pharmacologically acceptable salts thereof, with the proviso that the following compounds are not included:
*N*-[1-(4-morpholin-4-ylmethyl-phenyl)-3-pyridin-2-yl-4,5-dihydro-1*H*-pyrazolo[4,3-*g*]benzothiazol-7-yl]-acetamide,
*N*-[1-(4-dimethylaminomethyl-phenyl)-3-pyridin-3-yl-4,5-dihydro-1*H*-pyrazolo[4,3-*g*]benzothiazol-7-yl]-acetamide,
*N*-{1-[4-(benzylamino-methyl)-2-chloro-phenyl]-3-pyrazin-2-yl-4,5-dihydro-1*H-*pyrazolo[4,3-*g*]benzothiazol-7-yl}-acetamide and
*N*-(1-{2-chloro-4-[(1-cyclopentyl-piperidin-4-ylamino)-methyl]-phenyl}-3-pyrazin-2-yl-4,5-dihydro-1*H*-pyrazolo[4,3-*g*]benzothiazol-7-yl)-acetamide.

2. Compounds of general formula (1A), 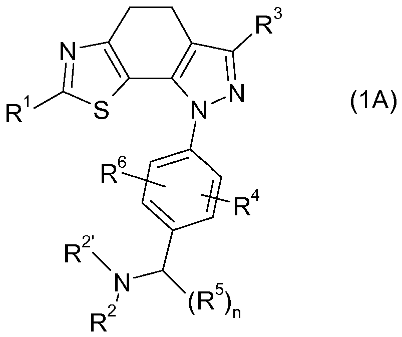 wherein the substituents are defined as in claim 1.

3. Compounds according to one of claims 1 or 2, wherein
**R³** denotes unsubstituted pyridyl.

4. Compounds according to one of claims 1 to 3, wherein
**R³** denotes unsubstituted pyridin-3-yl.

5. Compounds according to one of claims 1 to 4, wherein
**R¹** is selected from among -NHC(O)R^{a}, -NHC(O)OR^{a} and -NHC(O)NR^{a}R^{a}.

6. Compounds, or the pharmacologically acceptable salts thereof, according to one of claims 1 to 5, as medicaments.

7. Compounds, or the pharmacologically acceptable salts thereof, according to one of claims 1 to 5, for preparing a medicament with an antiproliferative activity.

8. Pharmaceutical preparations, containing as active substance one or more compounds of general formula (1) according to one of claims 1 to 5 or the pharmacologically acceptable salts thereof, optionally in combination with conventional excipients and/or carriers.

9. Use of compounds of general formula (1) according to one of claims 1 to 5 for preparing a medicament for the treatment and/or prevention of cancer.

10. Pharmaceutical preparation comprising a compound of general formula (1) according to one of claims 1 to 5 and at least one other cytostatic or cytotoxic active substance, different from formula (1), optionally in the form of the tautomers, the racemates, the enantiomers, the diastereomers and the mixtures thereof, as well as optionally the pharmacologically acceptable salts thereof.

## Revendications

1. Composés de formule générale (1) 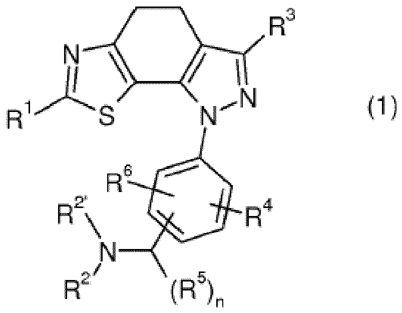 dans laquelle
R¹ est choisi dans le groupe comprenant -NHR^{a}, -NHC(O)R^{a}, -NHC(O)OR^{a}, -NHC(O)NR^{a}R^{a} et -NHC-(O)SR^{a}, et
R² et R^{2'} sont respectivement, indépendamment l'un de l'autre, un atome d'hydrogène ou un radical, choisi dans le groupe comprenant les groupes alkyle en C₁ à C₆, cycloalkyle en C₃ à C₈, aryle en C₆ à C₁₀, hétéroaryle de 5 à 10 chaînons et hétérocycloalkyle de 3 à 8 chaînons, éventuellement substitué par un ou plusieurs R^{a} et/ou R^{b}, ou
R² et R^{2'} conjointement avec l'atome d'azote inclus, forment un cycle hétérocycloalkyle ou hétéroaryle qui peut éventuellement contenir un ou plusieurs autres hétéroatomes choisis dans le groupe comprenant les atomes de N, O et S, et éventuellement substitué par un ou plusieurs R^{b} et/ou R^{d}, et
R³ est un radical choisi dans le groupe comprenant un groupe hétéroaryle de 5 à 6 chaînons, éventuellement substitué par un ou plusieurs R^{c} et/ou R^{b} identiques ou différents, et
R⁴ et R⁶ sont respectivement, indépendamment l'un de l'autre, un atome d'hydrogène ou un radical choisi dans le groupe comprenant un groupe halogène, -CF₃, -OCF₃, -CN, -NR^{c}R^{c}, -SR^{c}, -S(O)R^{c}, -S(O)₂R^{c} et -OR^{c} ou un groupe alkyle en C₁ à C₃ éventuellement substitué par un atome de fluor, un groupe -CN, -NR^{f}R^{f} et/ou -OR^{f}, et
R⁵ est un groupe alkyle en C₁ à C₃, ou
deux R⁵ forment, conjointement avec l'atome de carbone inclus, un cycle cycloalkyle en C₃ à C₈ ou un groupe hétérocycloalkyle de 3 à 8 chaînons et
n égal 0, 1 ou 2, ou
R² avec un R⁵ et avec l'atome d'azote et de carbone inclus forment conjointement un cycle hétérocycloalkyle de 4 à 8 chaînons, ou
R² avec un R⁶ approprié et les atomes d'azote et de carbone inclus forment conjointement un cycle hétérocycloalkyle de 4 à 8 chaînons, et chacun des R^{a}, indépendamment les uns des autres, est un atome d'hydrogène ou un radical substitué par un ou plusieurs R^{b} et/ou R^{c} identiques ou différents choisis dans le groupe comprenant un groupe alkyle en C₁ à C₆, cycloalkyle en C₃ à C₈, cycloalkylalkyle en C₄ à C₁₁, aryle en C₆ à C₁₀, arylalkyle en C₇ à C₁₆, hétéroaryle de 2 à 6 chaînons, hétérocycloalkyle de 3 à 8 chaînons, hétérocycloalkylalkyle de 4 à 14 chaînons, hétéroaryle de 5 à 10 chaînons et hétéroarylalkyle de 6 à 16 chaînons, et
chacun des R^{b} est un radical approprié choisi respectivement, indépendamment les uns des autres, dans le groupe comprenant =O, -OR^{c}, un groupe halogénoalkyloxy en C₁ à C₃, =S, -SR^{c}, =NR^{c}, =NOR^{c}, -NR^{c}R^{c}, halogéno, -CF₃, -CN, -NC, -NO₂, -N₃-S(O)R^{c}, -S(O)₂R^{c}, -S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{d})OR^{c}, -CN(R^{d})NR^{c}R^{c}, -OC-(O)R^{c}, -OC(O)OR^{c}, -OC(O)NR^{c}R^{c}, -OCN(R^{d})NR^{c}R^{c}, -N(R^{d})C(O)R^{c}, -N(R^{d})C(S)R^{c}, -N(R^{d})S(O)₂R^{c}, -N-(R^{d})C(O)OR^{c}, -N(R^{d})C(O)NR^{c}R^{c} et -N(R^{d})CN(R^{d})-NR^{c}R^{c}, et
chacun des R^{c}, indépendamment les uns des autres, est un atome d'hydrogène ou un radical substitué éventuellement par un ou plusieurs R^{d} identiques ou différents choisis dans le groupe comprenant les groupes alkyle en C₁ à C₆, cycloalkyle en C₃ à C₈, cycloalkylalkyle en C₄ à C₁₁, aryle en C₆ à C₁₀, arylalkyle en C₇ à C₁₆, hétéroaryle de 2 à 6 chaînons, hétérocycloalkyle de 3 à 8 chaînons, hétérocycloalkylalkyle de 4 à 14 chaînons, hétéroaryle de 5 à 10 chaînons et hétéroarylalkyle de 6 à 16 chaînons, et
chacun des R^{d}, indépendamment les uns des autres, est un atome d'hydrogène, un groupe alkyle en C₁ à C₆, cycloalkyle en C₃ à C₈, cycloalkylalkyle en C₄ à C₁₁, aryle en C₆ à C₁₀, arylalkyle en C₇ à C₁₆, hétéroaryle de 2 à 6 chaînons, hétérocycloalkyle de 3 à 8 chaînons, hétérocycloalkylalkyle de 4 à 14 chaînons, hétéroaryle de 5 à 10 chaînons et hétéroarylalkyle de 6 à 16 chaînons,
le terme "substituants alkyle" désignant respectivement des radicaux hydrocarbure aliphatiques saturés, insaturés, à chaîne linéaire ou ramifiée (radical alkyle) et comprenant non seulement les radicaux alkyle saturés mais aussi des radicaux alcényle et alcinyle insaturés ; et
le terme "cycloalkyle" désignant un cycle mono- ou bicyclique, le système cyclique pouvant être un cycle saturé mais également un cycle non aromatique insaturé qui peut contenir éventuellement des liaisons doubles ;
éventuellement sous la forme de leurs tautomères, leurs racémates, leurs énantiomères, leurs diastéréomères et leurs mélanges et éventuellement leurs sels pharmacologiquement acceptables à condition que les composés suivants ne soient pas compris :
N-[1-(4-morpholin-4-ylméthyl-phényl)-3-pyridin-2-yl-4,5-dihydro-1H-pyrazolo[4,3-g]-benzothiazol-7-yl]-acétamide,
N-[1-(4-diméthylaminométhyl-phényl)-3-pyridin-3-yl-4,5-dihydro-1H-pyrazolo[4,3-g]-benzothiazol-7-yl]-acétamide,
N-{1-[4-(benzylaminométhyl)-2-chloro-phényl]-3-pyrazin-2-yl-4,5-dihydro-1H-pyrazolo[4,3-g]benzothiazol-7-yl}-acétamide, et
N-(1-{2-chloro-4-[(1-cyclopentylpipéridin-4-yl-amino)-méthyl]-phényl}-3-pyrazin-2-yl-4,5-dihydro-1H-pyrazolo[4,3-g]benzothiazol-7-yl)-acétamide.

2. Composés de formule générale (1A), 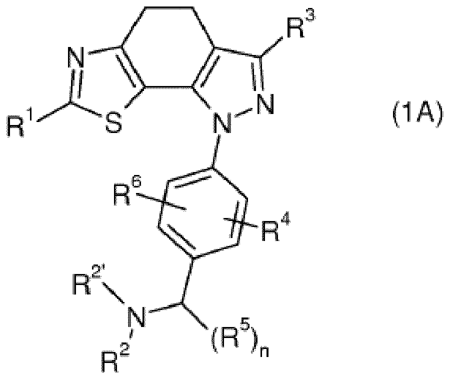 dans laquelle les substituants sont tels que définis dans la revendication 1.

3. Composés selon l'une quelconque des revendications 1 ou 2, dans lesquels
R³ désigne un groupe pyridyle non substitué.

4. Composés selon l'une quelconque des revendications 1 à 3, dans lesquels
R³ désigne un groupe pyridin-3-yle non substitué.

5. Composés selon l'une quelconque des revendications 1 à 4, dans lesquels
R¹ est choisi dans le groupe comprenant -NHC-(O)R^{a}_{,} -NHC(O)OR^{a} et -NHC(O)NR^{a}R^{a}_{.}

6. Composés ou sels pharmacologiquement acceptables, selon l'une quelconque des revendications 1 à 5, comme médicaments.

7. Composés ou sels pharmacologiquement acceptables, selon l'une quelconque des revendications 1 à 5, pour la production d'un médicament ayant un effet antiprolifératif.

8. Préparations pharmaceutiques contenant comme substance active, un ou plusieurs composés de formule générale (1) selon l'une quelconque des revendications 1 à 5, ou leurs sels pharmacologiquement acceptables, éventuellement en combinaisons avec des adjuvants et/ou véhicules habituels.

9. Utilisation de composés de formule générale (1) selon l'une quelconque des revendications 1 à 5, pour la production d'un médicament pour le traitement et/ou la prévention du cancer.

10. Préparation pharmaceutique comprenant un composé de formule générale (1) selon l'une quelconque des revendications 1 à 5, et au moins une autre substance active différente, cytostatique ou cytotoxique, de formule (1), éventuellement sous la forme de leurs tautomères, leurs racémates, leurs énantiomères, leurs diastéréomères et leurs mélanges, et leurs sels pharmacologiquement acceptables.
